# EUROPEAN PATENT SPECIFICATION

(11) **EP 2 914 284 B1**
(45) Date of publication and mention of the grant of the patent: **21.09.2016**
(21) Application number: 13789738.5
(22) Date of filing: 30.10.2013
(51) Int. Cl.: A61K 39/245

(54) **RECOMBINANT PARTICLE BASED VACCINES AGAINST HUMAN CYTOMEGALOVIRUS INFECTION**
REKOMBINANTE IMPSTOFFE AUF BASIS VON PARTIKELN GEGEN EINE INFEKTION MIT DEM HUMANEN CYTOMEGALOVIRUS
VACCINS RECOMBINANTS À BASE DE PARTICULES CONTRE L'INFECTION PAR LE CYTOMÉGALOVIRUS HUMAIN

(30) Priority: 30.10.2012 EP 12190652
(43) Date of publication of application: 09.09.2015
(73) Proprietor: Pfizer Inc., New York, NY 10017-5755 (US)
(72) Inventor: WELLNITZ, Sabine, CH-8902 Urdorf (CH); JOHN, Corinne, CH-8810 Horgen (CH); SCHAUB, Christian, CH-8820 Wädenswil (CH)
(74) Representative: Schiweck, Weinzierl & Koch
(86) International application number: PCT/EP2013/072717
(87) International publication number: WO 2014/068001

(56) References cited:
- WO-A1-2008/138590
- WO-A1-2010/125201
- WO-A2-2010/128338
- TANG XIAN-CHUN ET AL: "Baculovirus-Produced Influenza Virus-like Particles in Mammalian Cells Protect Mice from Lethal Influenza Challenge", VIRAL IMMUNOLOGY, vol. 24, no. 4, August 2011 (2011-08), pages 311-319, XP002711212,

## Description

### Field of the Invention

The invention relates to virus-like particles comprising capsid proteins, cytomegalovirus surface proteins, and optionally tegument proteins, and vaccines against human cytomegalovirus infection.

### Background Art

Human cytomegalovirus (HCMV) is found in all parts of the world and in all socioeconomic groups. It infects at least 60% of the adult population; in some countries a 100% endemic infection is already reached. Usually infection is asymptomatic in immunocompetent individuals; at times, a mononucleosis-like illness can occur. The infection invariably leads to the establishment of lifelong latency, which may occasionally be interrupted by reactivation. Immunodeficient individuals, such as therapeutically or iatrogenically immunosuppressed transplant/cancer patients, neonates, and HIV patients, present significant morbidity and mortality as a result of HCMV infection.

The current therapy of CMV infection is treatment with antiviral reagents such as ganciclovir (valganciclovir), foscarnet, acyclovir, cidofovir or leflunomide. Application of such antiviral agents has been approved for transplant recipients and immuno-compromised patients (e.g. HIV patients), but not in pregnant women. For these a hyper-immunoglobin therapy is preferred. Additionally, the existing antiviral therapies lead to viral resistance in a short timeframe or were not effective during therapy (McGregor A. et al., Expert Opin. Drug Metab. Toxicol. 2011; 7(10):1245-1265). Although prevention of cytomegalovirus infection, especially for congenital infection has been investigated for a long time, there is no licensed vaccine on the market. The development of a prophylactic HCMV vaccine has been identified as a high priority goal by the Institute of Medicine in the USA (www.cdc.gov/cmv/overview.html, October 2011). The potential benefit from vaccine-induced immunity has been estimated to be 40-fold with respect to intra-uterine transmission and 25- to 30-fold with respect to decrease in central nervous system damage in congenitally infected infants (Britt W.J. et al., Trends Microbiol. 1996; 4:34-38). There have been efforts made for over 30 years to develop vaccines. However, so far no imminent licensure appears likely, due to missing or incomplete clinical study data. Neither the live attenuated virus approaches nor the attempts with soluble proteins, especially with the truncated gpUL55 (gB) subunit vaccine in combination with or without an adjuvant like MF59, led to a long-lasting immune response (Zhang C. et al., J. Clin. Virol 2006; 35:338-41). New approaches to reach higher efficacy are under investigation and comprise glycoprotein combinations, DNA and peptide based candidates, dense bodies (WO 2008/138590) and viral vector based candidates such as the replication competent Alvac-gpUL555 (gB) vaccine based on a canarypox virus (Bernstein D.I. et al., J. Infect. Dis. 2002; 185:686-90). The first data of clinical trials with the Alvac vaccine (canarypox based particle) in combination with a live attenuated virus led to an appropriate immune response whereas the long-lasting effects are still under investigation. Virus-like particles can accommodate properties similar to attenuated viruses due to their shape and are therefore a promising option for vaccine development, especially since they are generally accepted as excellent vaccine vehicles. Virus-like particles have not been developed for CMV up to now. On the basis of the experience gained over the last 30 years for the development of an anti-HCMV vaccine, the following goals should be met: a long-lasting induction of neutralizing antibodies, induction of humoral and cellular, especially cytotoxic T-cell responses and minimizing side effects.

The reasons for failure or limited efficacy of vaccine candidates developed so far are multifaceted. The mechanisms of protective immunity in a host are poorly elucidated. One of the main reasons for this is the lack of an appropriate animal model to be able to verify the effectors accounting for protection. The ability of the virus to enter and replicate in diverse cell types including epithelial cells, endothelial cells, smooth muscle cells, fibroblasts, neurons and monocytes/macrophages resulting in different pathologies hinders the determination of potent effectors. Due to the complexity of the virus, and the lack of knowledge concerning proteins required for infection of specific cell types, the composition of an ideal HCMV vaccine is so far uncertain.

CMV is a beta-herpesvirus and belongs to the very complex viral order *Herpesvirales.* The CMV virion is -230 nm in diameter and is composed of a nucleocapsid, surrounded by a less structured tegument layer, and bounded by a trilaminate membrane envelope. The linear, double-stranded DNA molecule (236 kbp) is the largest among the human herpesviruses and over 50% larger than that of Herpes simplex virus 1 (HSV-1). In addition to virions, five other types of intracellular, enveloped and non-enveloped virus particles have been recovered from CMV-infected cells. The virus maturation process includes different capsid types of which dense bodies (DB) may play an important role in vaccine development. Dense bodies (DBs) are large (-250-600 nm) and heterogeneous. They are solid spheroidal aggregates of different proteins containing a tegument protein (i.e. pUL83) and the surface proteins gpUL75 (gH) and gpUL55 (gB) (Becke S. et al., Vaccine 2010; 28:6191-6198). Uniform nomenclature rules for HCMV proteins were published in Spaete R. et al., 1994, J. Gen. Virol., 75, 3287-3308: an alphanumeric designation of each identified open reading frame (ORF) is the basis, extended by the prefixes "p" if the phosphorylation status of the protein is not known, "pp" for phosphoproteins and "gp" for glycoproteins. Small letter designations are used as suffixes to name additional ORFs that encode proteins. In the present invention the previously used designations are indicated in brackets, and are also indicated in tables and figures.

Among the HCMV structural glycoproteins, three major complexes (designated as gCl-gCIII) have been identified so far. These complexes play different roles in the virus structure and thus also for the design of vaccines, as targets for the development of therapeutic antibodies, or as research tools. The glycoprotein B (gpUL55, gCl) as a single soluble protein did not lead to a long lasting immune response against HCMV. Therefore, further protein compositions including gpUL55 should be taken into account for vaccine design. The role of gpUL100 (gM), gpUL73 (gN) and the formed complex gcII for generation of neutralizing antibodies and as an essential part of a vaccine is under investigation. Constituents of gCIII are gpUL75 (gH), gpUL115 (gL) and gpUL74 (gO) forming a heterotrimeric, disulfide-linked complex (Huber M.T. and Compton T., J. Virol. 1999; 73(5):3886-3892). These proteins play the most important role for viral entry and complex formation with further proteins such as gpUL128, gpUL130 and gpUL131A. The entry of HCMV into epithelial and endothelial cells involves endocytosis and complex formation of gpUL75, gpUL115 and gpUL74, or of the combination of gpUL75, gpUL115, gpUL128, gpUL130 and gpUL131 (Wang D. and Shenk T., PNAS 2005; 102:18153-18158). In addition these proteins are responsible for the transfer of HCMV from endothelial cells to leukocytes, representing major determinants of HCMV dissemination *in vivo.* Furthermore, these proteins seem to play a role in the priming of dendritic cells (DC) and are therefore critical elements for T-cell mediated immune response. CMV enters different cell types by distinct pathways. For fibroblast entry gpUL75/gpUL115 (gH/gL) or gpUL75/gpUL115/gpUL74 (gH/gL/gO) containing complexes are required. This is a pH-independent, non-endosomal infection pathway which could be blocked by neutralizing antibodies generated after vaccination with a Towne strain or gpUL55 (gB) adjuvant (MF59) based vaccine (Pass R.F., J. Clin. Virol, 2009; 46 (Suppl. 4), S73-76).

The second important pathway is an endosomal infection process of epithelial and endothelial cells which requires the pentameric complex gpUL75/ gpUL115/ gpUL128/ gpUL130/ gpUL131 (gH/gL/UL128/UL130/UL131A). This infection route can be blocked by neutralizing antibodies targeting the pentameric complex (Manley K. et al., Cell Host & Microbe 2011; 10:197-209). From the current state of knowledge different combinations of the above proteins are required to generate an efficient vaccine that stimulates a broad cellular and humoral immune response. However, the exact combination is not known. The importance of the proteins of the pentameric complex was addressed recently by Lilleri D. et al., PLoS One, March 2013 ; 8(3): e59863, 1-13.

Virus-like particles (VLPs) are stable, highly organised spheres that self-assemble from virus-derived structural antigens and thus have structural characteristics and antigenicity similar to the parental virus. In contrast to live attenuated viruses, the recombinant VLPs are safe and non-infectious since they do not contain replicating viral DNA. Therefore, they can be generated under biosafety level 1.

Several different viral vectors like adenoviral, retroviral, canarypox, vaccinia (poxvirus) and modified vaccinia virus ankara (MVA) vectors were so far used to express foreign antigens for pharmaceutical development as vaccines (Wang Z. et al., J. of Virology 2004; 3965-3976; Jie Zhong et al., Plos One 2008; 3(9):e3256). Most of the above mentioned vectors are replication competent, especially the retrovirus based vectors (Dalba C. et al., Mol. Ther. 2007; 15(3):457-466). Recombinant VLPs however do not have any risks associated with virus replication or inactivation as other vaccine types have. VLPs provide, on the basis of their particulate nature, an inherent advantage over soluble antigens in respect to immunogenicity and stability. Compared to subunit or single recombinant proteins, VLPs are more immunogenic and are able to stimulate B-cell mediated immune responses as well as CD4 proliferative and CTL responses (Ludwig C. and Wagner R., Curr. Opin. Biotechnol. 2007; 18:537-545). The size of VLPs appears to be favourable for uptake by dentritic cells (DC) through endocytosis or macropinocytosis and activating innate and adaptive immune response. VLPs, especially retroviral VLPs (gag-VLPs), contain immunogenic epitopes and are supposed to stimulate cellular immune responses via MHC class-I and MHC class-II pathways (DemI L. et al., Mol. Immunol. 2005; 42:259-277). Because of these immunogenic properties VLPs do not appear to require the use of adjuvants to achieve potent immune stimulation. VLPs have been extensively, and quite successfully, used as vaccine (WO 2005/123125) and viral serology reagents.

For enveloped viruses, VLPs require at least one capsid or matrix protein for the assembly of a viral particle. The proteins assemble in different cellular compartments such as endoplasmic reticulum (ER), lipid rafts or plasma membrane where the budding takes place, and thus contain the cellular lipids building the viral lipoprotein envelope. The VLPs may also include host cell proteins, e.g. lipid raft associated gangliosides. VLPs can be exploited for presentation of foreign epitopes and/or targeting molecules based on single or multicomponent capsids. The core gene of hepatitis B virus fused with antigens provided an early example of the VLP approach (WO 1999/057289). To date no recombinant CMV VLP has been generated. The largest VLPs generated and published are Influenza, Bluetongue and Rotavirus VLPs that contain up to 5 different proteins. It is not exactly known how many and which proteins are necessary to form a recombinant CMV particle based on CMV capsid proteins. In contrast, many non-herpesvirus capsid proteins are known to form VLPs. The most prominent example is the retroviral precursor protein gag, in particular MoMuLV (Moloney Murine Leukemia Virus), HIV (Human Immunodeficiency virus), HTLV (Human T-Cell Leukemia Virus) and SIV (Simian Immunodeficiency Virus) gag. The pr55 HIV precursor protein forms a VLP with a size of 100-120 nm and was shown to serve as carrier for further proteins The MoMuLVprecursor protein gag (pr65) leads to VLPs with a size of 115-150 nm in a high yield based on immunoblotting and ELISA assays.

The baculovirus expression vector system (BEVS) is highly suitable for the co-infection and co-expression of proteins for the production of vaccines or other biologics, since its genome and virus structure allows for large, foreign gene insertions. It is safe due to the narrow host range, restricted primarily to Lepidopteran species (moths and butterflies). The primary production host are insect cells, however, the baculovirus can also be applied for recombinant co-expression in mammalian cells. The baculovirus system is a widely used and highly efficient system for protein expression in research and commercial laboratories around the world (Roldao A. et al., Expert Rev. Vaccines, 2010; 9(10):1149-1176). GlaxoSmithKline's human papillomavirus (HPV) VLP vaccine is produced in BEVS and approved for use in the USA and the European Union (WO 2005/123125).

WO 2010/128338 discloses CMV vaccines comprising Herpes Simplex Virus (HSV) or a HSV virus-like particle (HSV VLP) further comprising a Human Cytomegalovirus (HCMV) polyepitope.

### Summary of the Invention

The invention relates to a recombinant virus-like particle comprising one or more capsid or capsid precursor proteins, 3, 4, 5 or more different surface proteins from cytomegalovirus (CMV), and optionally one or more tegument proteins.

Preferably the capsid proteins are derived from a herpes virus such as cytomegalovirus, e.g. human cytomegalovirus (HCMV), or from a retrovirus, in particular HIV or MoMuLV. Preferred capsid protein is the HIV and/or MoMuLV precursor gag protein.

The surface proteins (also termed envelope proteins) are preferably selected from HCMV, in particular from the group consisting of gpUL75 (gH), gpUL115 (gL), gpUL55 (gB), gpUL74 (gO), gp100 (gM), gp73 (gN), gpUL128, gpUL130, and gpUL131A. Likewise the tegument proteins are preferably selected from HCMV, in particular from the group consisting of pUL32, pUL45, pUL47, pUL48, pUL69, pUL71, pUL72, pUL76, pUL77, pUL83 (pp65), pUL88, pUL93, pUL94, pUL95, pUL97, pUL99, and pUL103. The core proteins are in particular selected from the group consisting of pUL23, pUL24, pUL33, pUL36, pUL38, pUL43, pUL78, pUL82, pUL96, IRS1, US22, and TRS1 having either tegument or envelope protein character. Likewise the regulatory proteins are preferably selected from the group of IE-1, UL50, UL80.5, UL46 and UL47.

The recombinant virus-like particle according to the invention may further comprise B- and/or T-cell epitopes, proteins selected from the group consisting of additional foreign antigenic sequences, cytokines, CpG motifs, g-CMSF, CD19, and CD40 ligand, and/or fluorescent proteins, proteins useful for purification purposes of the particles or for attaching a label, and/or proteinaceous structures required for transport processes.

Furthermore the invention relates to a DNA encoding the proteins comprised in the virus-like particles according to the invention, to a vector comprising such DNA, in particular a baculovirus vector, and a host cell comprising such a vector, and to methods of manufacturing virus-like particles according to the invention using a baculovirus vector.

Furthermore the invention relates to a vaccine comprising a recombinant virus-like particle according to the invention, in particular to such a vaccine further comprising the pentameric complex consisting of gpUL75, gpUL115, gpUL128, gpUL130 and gpUL131A, and/or soluble CMV protein selected from the group consisting of gpUL75, gpUL115, gpUL55, gpUL74, gpUL100, and gpUL73; or gpUL128, gpUL130, and gpUL131A; or pUL83, IE-1, UL99, UL91, and pp150. Such a vaccine may further comprise an adjuvant selected from the group consisting of aluminium hydroxide, alum, AS01, AS02, AS03, AS04, MF59, MPL, QS21, ISCOMs, IC31, unmethylated CpG, ADVAX, RNA containing formulations and Freund's reagent, but also DNA of the invention as defined above.

In a particular embodiment, the vaccine comprises CMV proteins from different CMV strains selected from the group of Towne, Toledo, AD169, Merlin, TB20, and VR1814 strains.

The vaccine comprising a recombinant virus-like particle according to the invention may be further enhanced in its induction of host immune response by a second immunization with the pentameric complex consisting of gpUL75, gpUL115, gpUL128, gpUL130, and gpUL131A in a prime-boost administration, and/or with a soluble CMV protein selected from the group consisting of gpUL75, gpUL115, gpUL55, gpUL74, gpUL100, gpUL73, pUL83, and IE-1 in a prime-boost administration.

Alternatively, the vaccine comprising a recombinant virus-like particle according to the invention may be further enhanced in its induction of host immune response (humoral and cellular response) by addition of the soluble complexes consisting of at least two different surface proteins out of the group consisting of gpUL73, gpUL74, gpUL75, gpUL100, gpUL115, gpUL128, gpUL130, and gpUL131A.

### Brief Description of the Figures

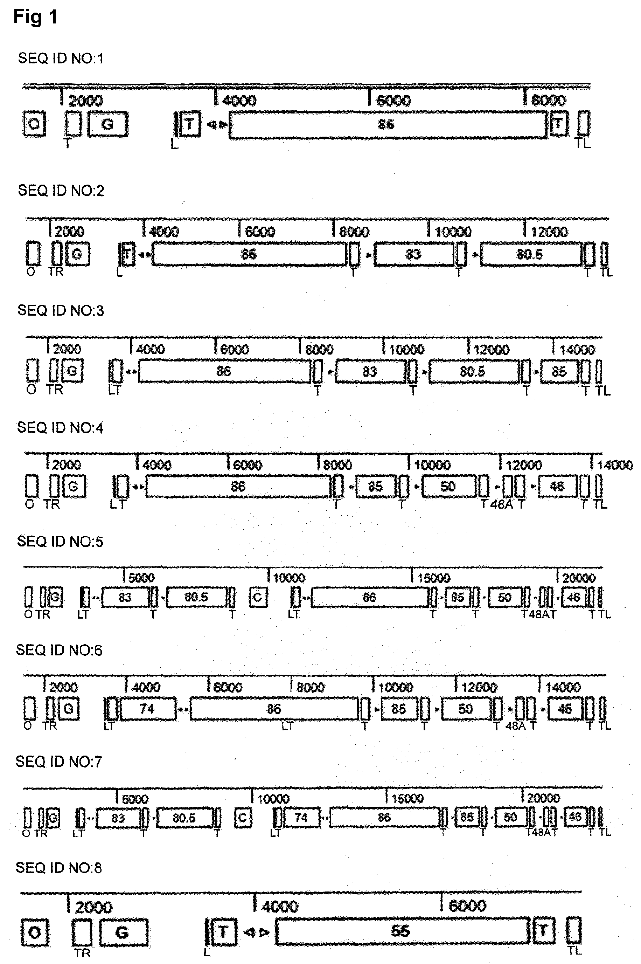

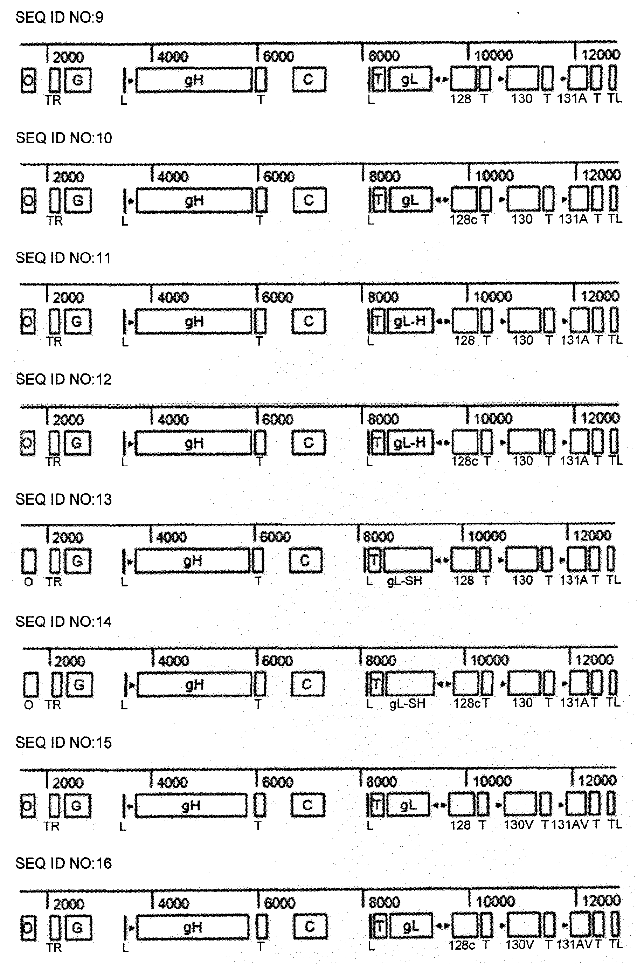

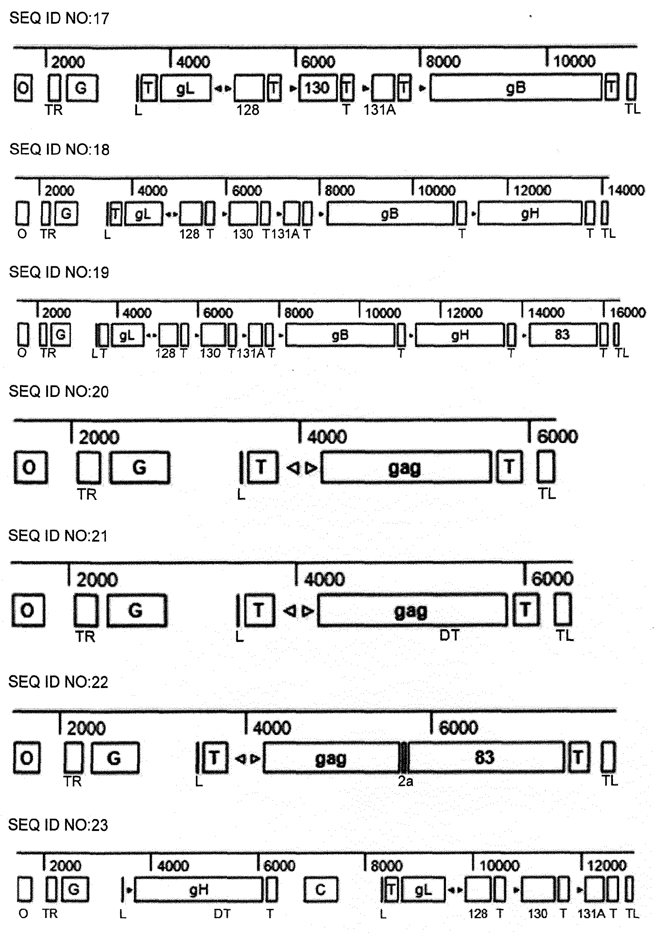

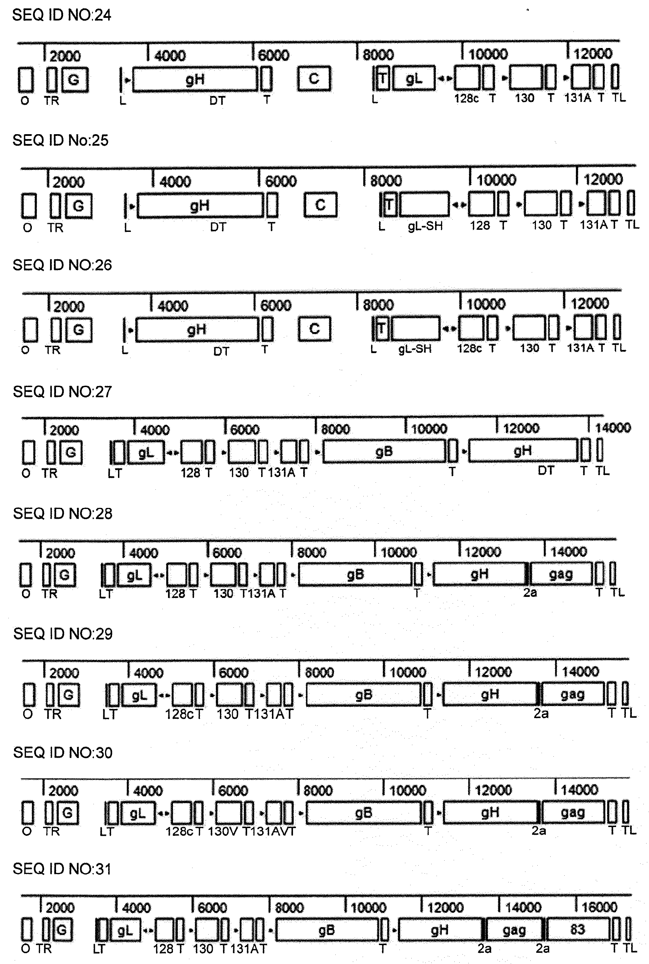

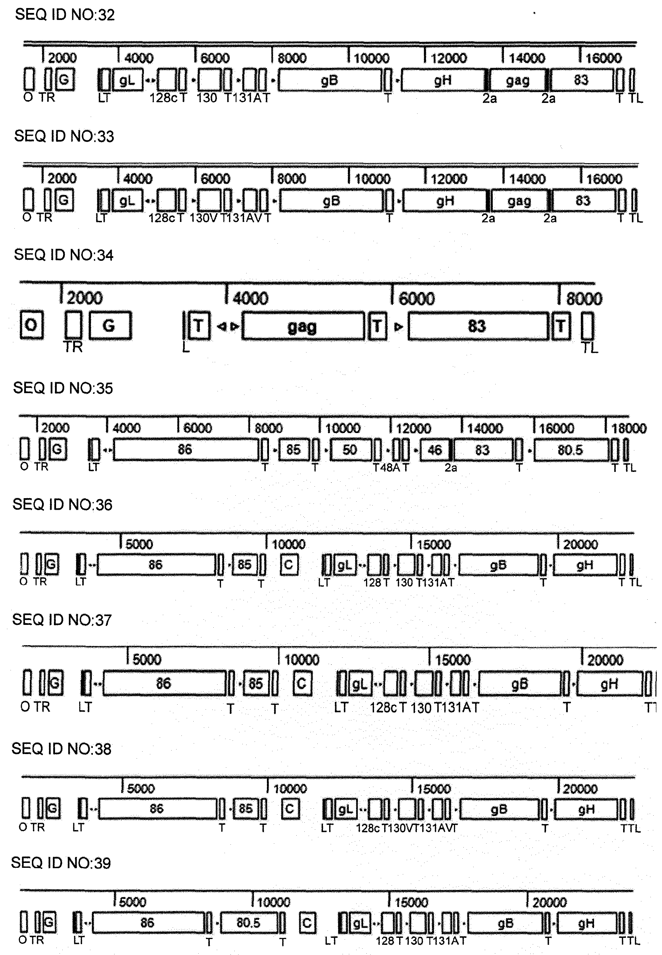

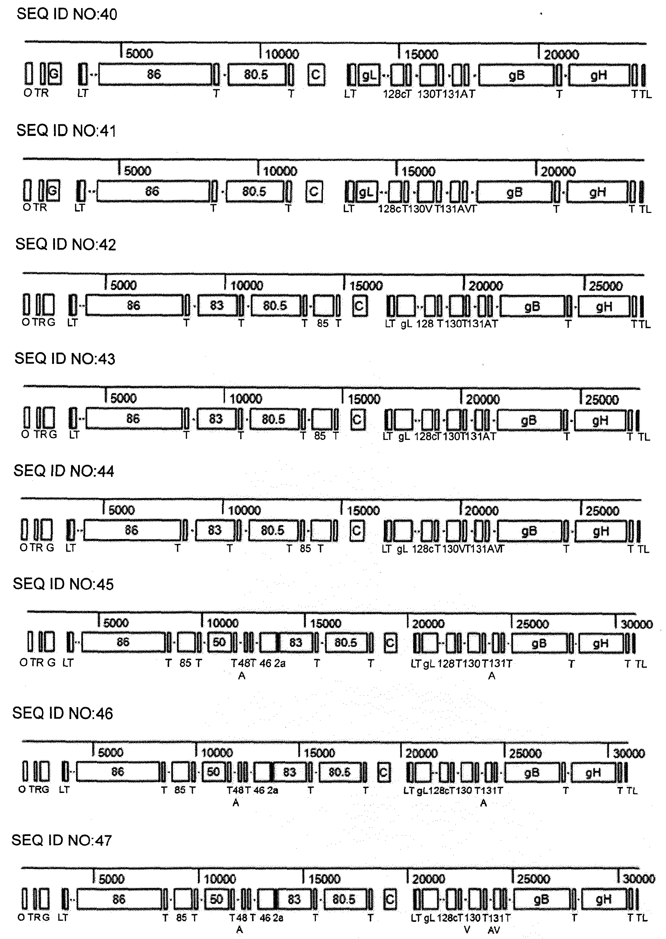

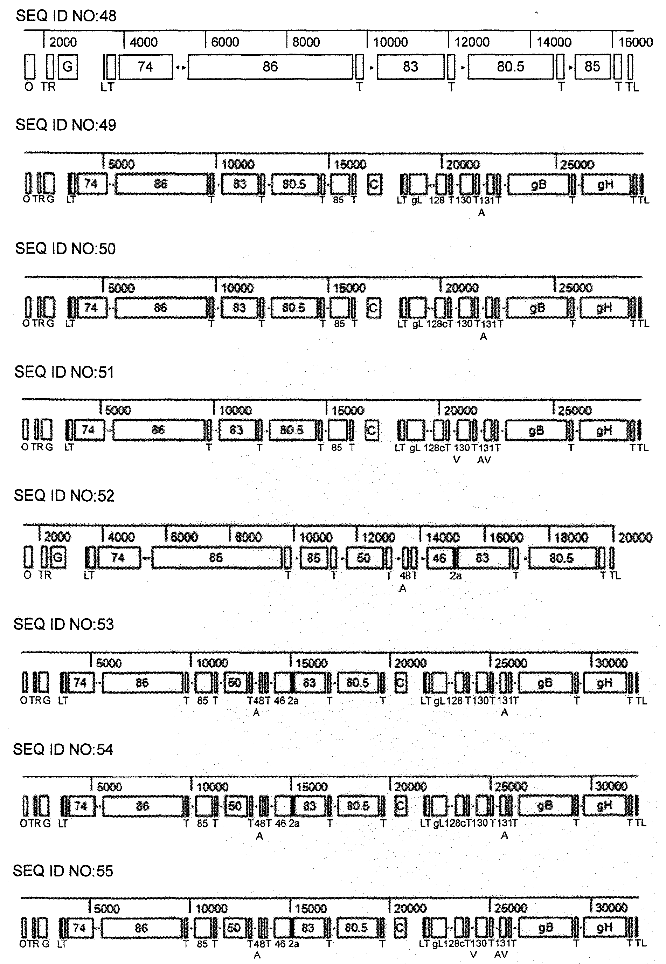

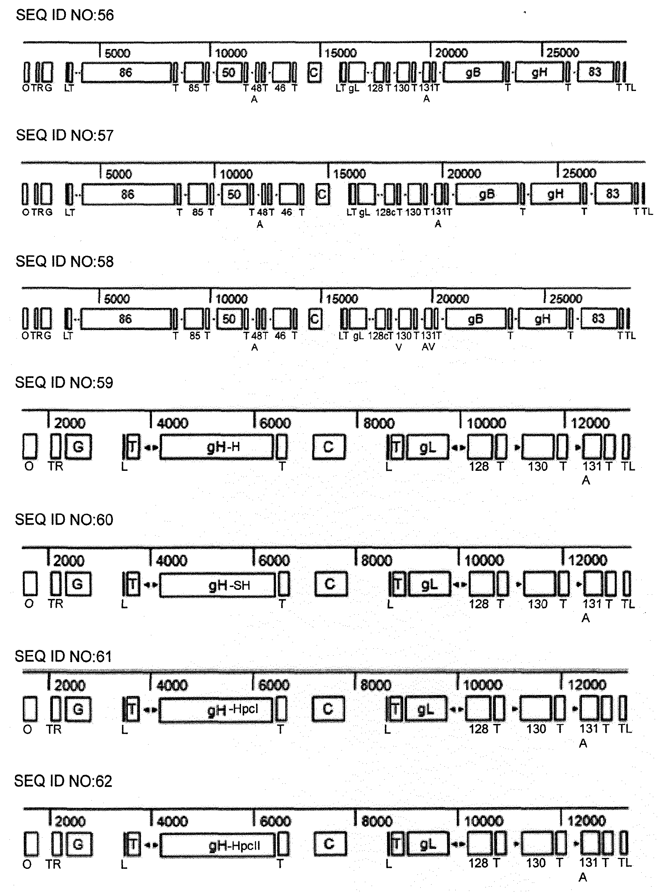

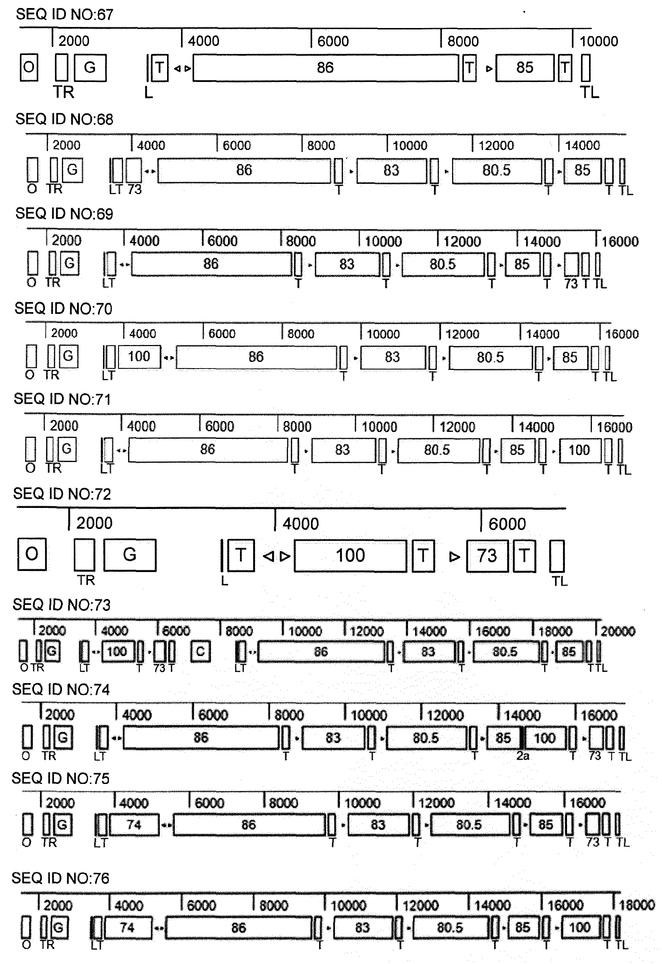

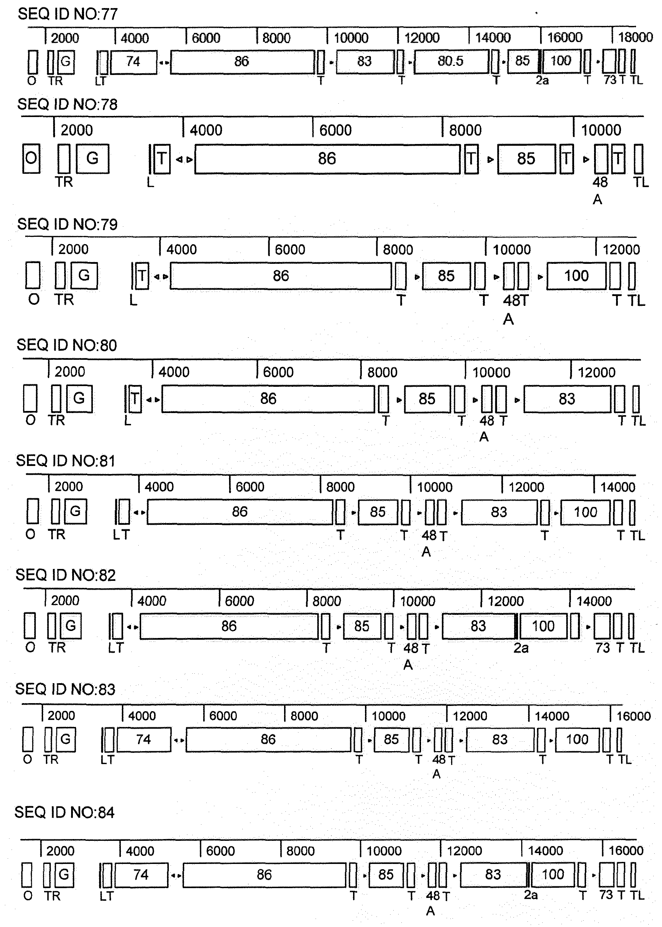

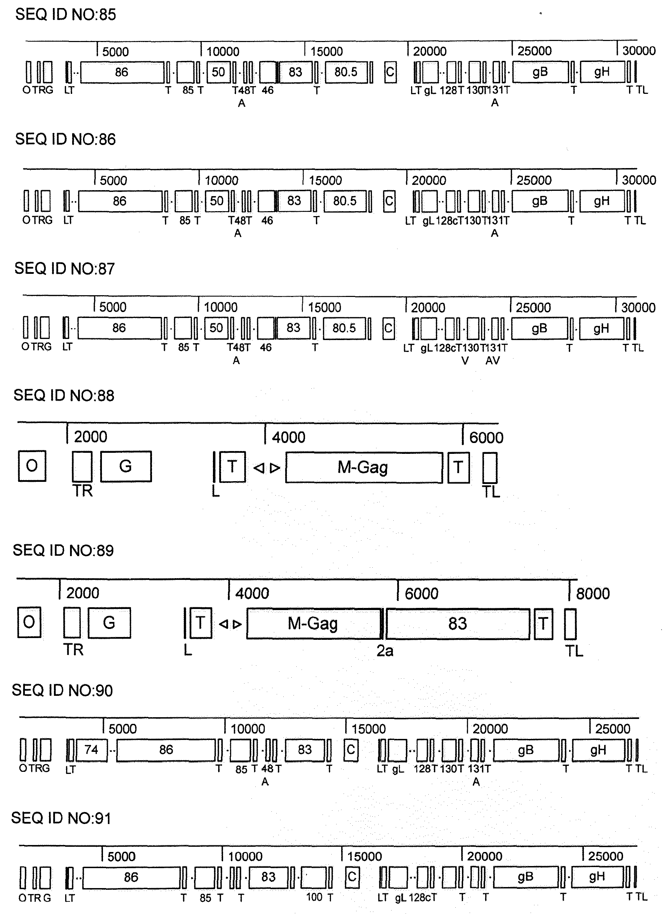

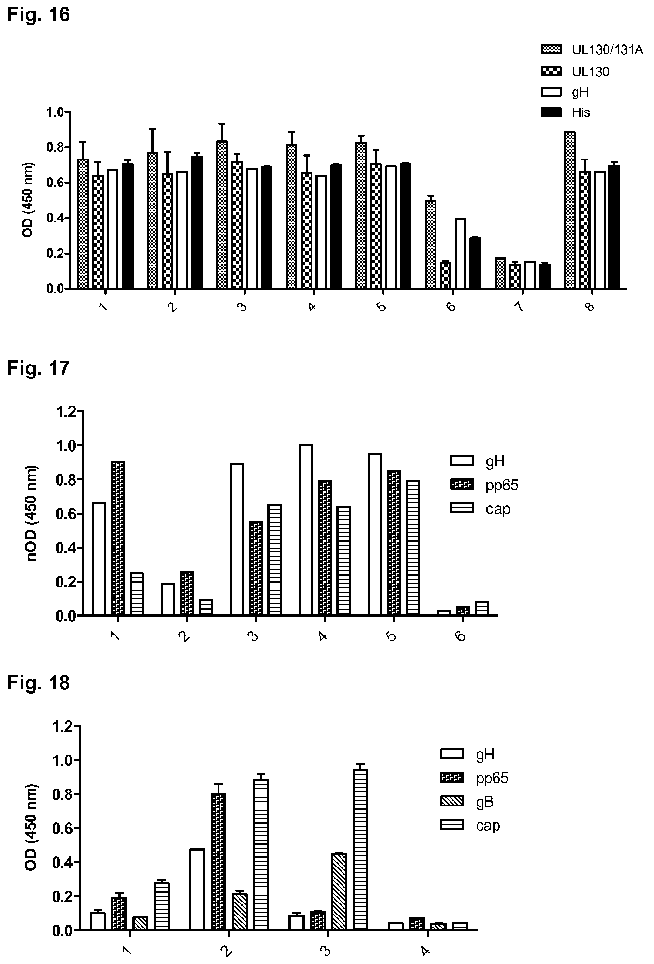

*Figure 1**: Schematic representations of recombinant vectors for expression of different variants of CMV virus-like particles, either based on a herpesvirus or non-herpesvirus capsid, or for expression of CMV-pentameric complex and soluble CMV proteins.*
   The different variants are inserted into the vector backbone pRBT136 aimed at recombinant protein expression using the baculovirus expression system (BEVS) and containing two promoters P1 and P2 (◁_p10, ▷_polh) and two terminator sequences T1 and T2 (T), which are SV40 and HSVtk. For propagation in yeast the vectors contain an origin of replication (O), e.g. 2micron, and a marker gene (m), e.g. URA3. Furthermore the vectors contain the transposon sites left (TL) and right (TR) for transposition of the transgenes from the transfer vector into bacmids, a IoxP site (L) for site specific homologous recombination (plasmid fusion), origins of replication (O), ampicillin (A), chloramphenicol (C) and gentamycin (G) resistance genes, and defined restriction sites. For the expression in mammalian cells, either by transduction with a baculovirus or transient expression, the vector backbone pRBT 393 contains in addition a promoter selected from pCMV, ie1 and lef2, and a terminator selected from SV40pA, BHG pA and HSVtk.
   Abbreviations: c: consensus sequence; H: His-tag; SH: Streptavidin-His-tag; V: strain VR1814, pcl: precission protease, pcll: precission and TEV protease, DT: dimerization tool, T: terminator, O: origin of replication, G: gentamycin resistance, C: chloramphenicol resistance, L: IoxP site, TL: left transposon side, TR: right transposon side. The previous, shorter HCMV nomenclature (gB, gH, gL, gO as well as "UL" without prefix and UL48 without suffix "A") is used. Genes are labelled only with their numbers, e.g. "83" instead of "UL83".

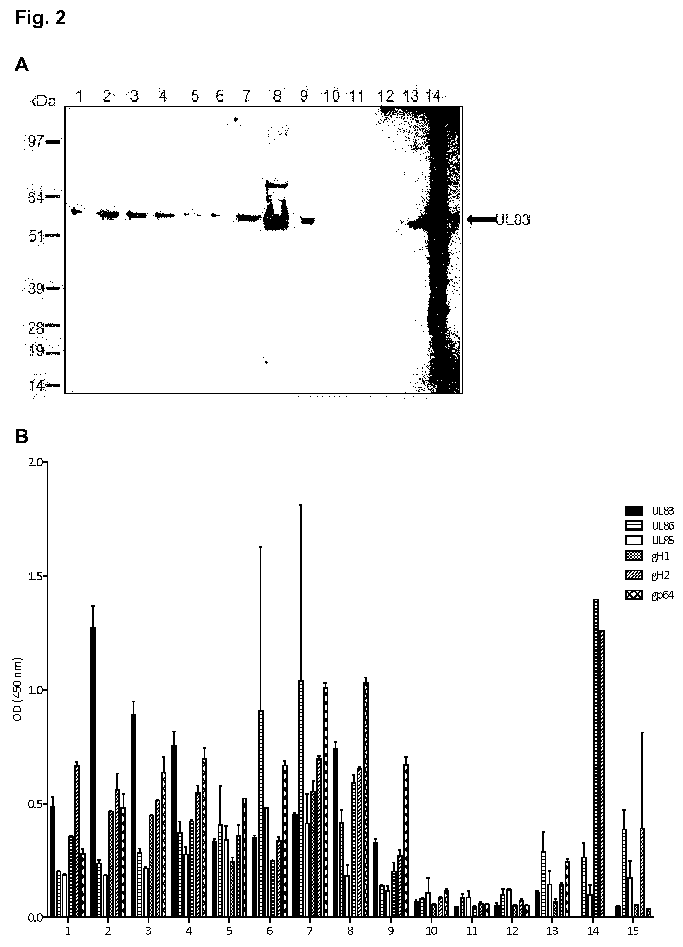

*Figure 2**: Glycerol-tartrate gradient based purification of a CMV-VLP variant SEQ ID NO:7 combined with SEQ ID NO:14.*
   (A) Glycerol-tartrate gradient based purification. Analysis of different fractions of CMV-VLP variant comprising the capsid, functional, tegument and surface proteins UL86-UL85-UL50-UL48A-UL46-UL74(gO)-UL83-UL80.5-UL75(gH)-UL115(gL)-UL128(c)-UL130-UL131A (SEQ ID NO: 7 and SEQ ID NO:14) from the Towne strain by SDS-PAGE (4-12% Bis-Tris gel) followed by immunoblotting. A mouse anti-pUL83 (Virusys) antibody was used for verification of the tegument protein. On the left side a protein standard in kDa is shown; lanes 1 to 13: fractions 1 to 13 (top to bottom fractionation), lane 14: positive control.
   (B) Verification of the immunoblot data by ELISA assay using specific antibodies against capsid (UL86, UL85), tegument (UL83) and surface UL75 (gH1, gH2) proteins. Presence of two capsid, a tegument protein (UL83) and surface protein UL75 (gH) could be shown by antibody binding to the selected proteins (UL86, UL85, gH2: University of Alabama, pUL83: Virusys; gH1: Santa Cruz). The presence of baculoviruses was verified with an antibody against the protein gp64 (eBioscience). Number 1-13 of the x-axis represents the number of the glycerol-tartrate gradient (fractionated from top to bottom), number 14 represents a positive and number 15 a negative control. Binding strength of antibody to protein in the VLPs is shown as optical density (OD) at the y-axis. Detection of the selected proteins in the same fractions showed their co-localization and therefore intact CMV-VLPs. Similar results were obtained for the expression and purification of the variants SEQ ID NO:7 combined with SEQ ID NO:9, SEQ ID NO:10 and SEQ ID NO:13.

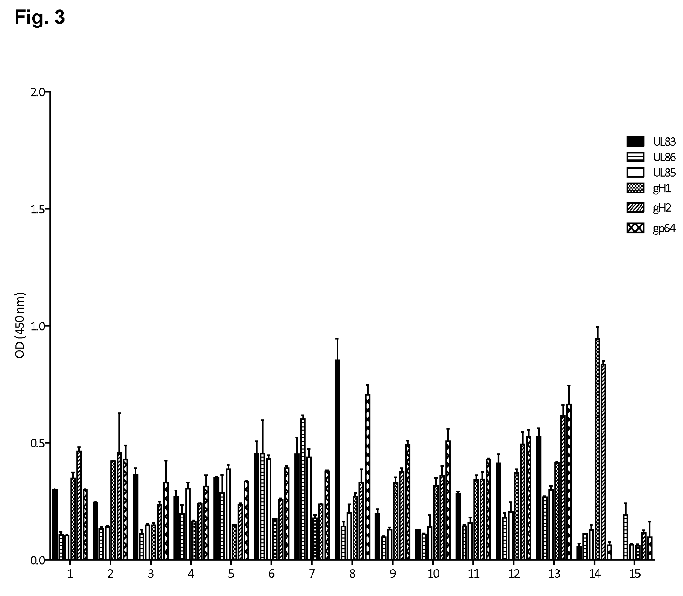

*Figure 3**: Glycerol-tartrate gradient based purification of a CMV-VLP variant SEQ ID NO:5 combined with SEQ ID NO:14.*
   Verification of immunoblot data by ELISA assay using specific antibodies against the capsid proteins UL86 and UL85, against the tegument protein UL83 (UL83) and surface protein UL75 (gH). Presence of capsid (UL86, UL85), tegument (UL83) and surface protein UL75 (gH) could be shown by antibody binding to the selected proteins (UL86, UL85, gH1: antibody provided by University of Alabama, UL83: Virusys; gH2: Santa Cruz). The presence of baculoviruses was verified with an antibody against the protein gp64 (eBioscience). Number 1-13 of the x-axis represents the number of the glycerol-tartrate gradient (fractionated from top to bottom), number 14 represents a positive and number 15 a negative control. Binding strength of antibody to protein in the VLPs is shown as optical density (OD) at the y-axis. Detection of the selected proteins in the same fractions showed their co-localization and therefore intact CMV-VLPs.
   The CMV VLPs composed of SEQ ID NO:5 and SEQ ID NO: 13 led to nearly identical data. To verify the composition of the surface proteins the CMV VLPs generated on the basis of SEQ ID NO: 5 combined with SEQ ID NOs: 9 or 10 were treated in the above mentioned way and showed similar OD values within a range of 0.1-0.3.

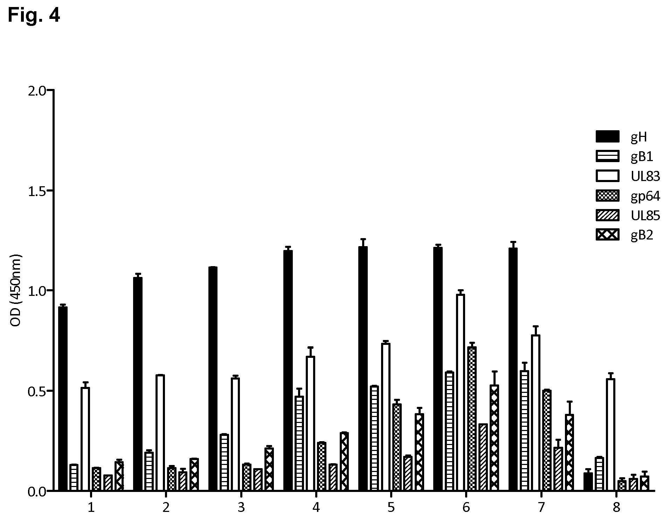

*Figure 4**: Glycerol-tartrate gradient based purification of a CMV-VLP variant SEQ ID NO:3 combined with SEQ ID NO:18.*
   Verification of immunoblot data by ELISA assay using specific antibodies against capsid protein (UL85), tegument (UL83) and surface (UL75 (gH), UL55 (gB)) proteins. Presence of capsid (UL85), tegument (pUL83) and surface proteins (gpUL75, gpUL55) could be shown by antibody binding to the selected proteins (UL85/UL55 (gB1): University of Alabama; UL83/UL55 (gB2): Virusys; UL75 (gH): Santa Cruz). Number 1-7 of the x-axis represents the number of the glycerol-tartrate gradient fraction 1 to 7 (fractionated from top to bottom), number 8 represents a negative control. Binding strength of antibody to protein in the VLPs is shown as optical density (OD) at the y-axis. Detection of the selected proteins in the same fractions showed their co-localization and therefore intact CMV-VLPs. The same intact composition was observed by combining SEQ ID NO:3 with SEQ ID NO:9, 10, 16 or 19 where the OD value detection differs in a range between 0.1-0.4.

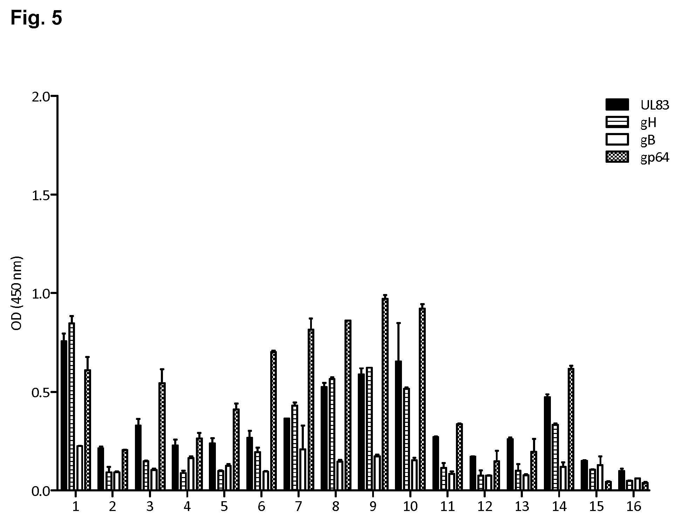

*Figure 5**: Glycerol-tartrate gradient based purification of a CMV-VLP variant SEQ ID NO:2 combined with SEQ ID NO:18.*
   Verification of immunoblot data by ELISA assay using specific antibodies against tegument (UL83) and surface (UL75, UL55) proteins. Presence of a tegument protein (UL83) and surface protein (UL75, UL55) could be shown by antibody binding to the selected proteins (UL83/UL55 (gB): Virusys; UL75 (gH): Santa Cruz). The presence of baculoviruses was verified with an antibody against the protein gp64 (eBioscience). Number 1 of the x-axis represents the sample of the pre-purification by sucrose cushion, lanes 2-14 of the x-axis represents the number of the glycerol-tartrate gradient fractions 1 to 13 (fractionated from top to bottom), number 15 represents a negative control for VLPs and number 16 a negative control for baculoviruses. Binding strength of antibody to protein in the VLPs is shown as optical density (OD) at the y-axis. Detection of the selected proteins in the same fractions showed their co-localization and therefore intact CMV-VLPs. The same proteins as mentioned above could be verified as well in the CMV VLP variants composed of SEQ ID NO: 2 for the capsid and tegument and SEQ ID NO: 9, 10, 16 or 19 for the surface proteins in a range of 0.1-0.3 OD values.

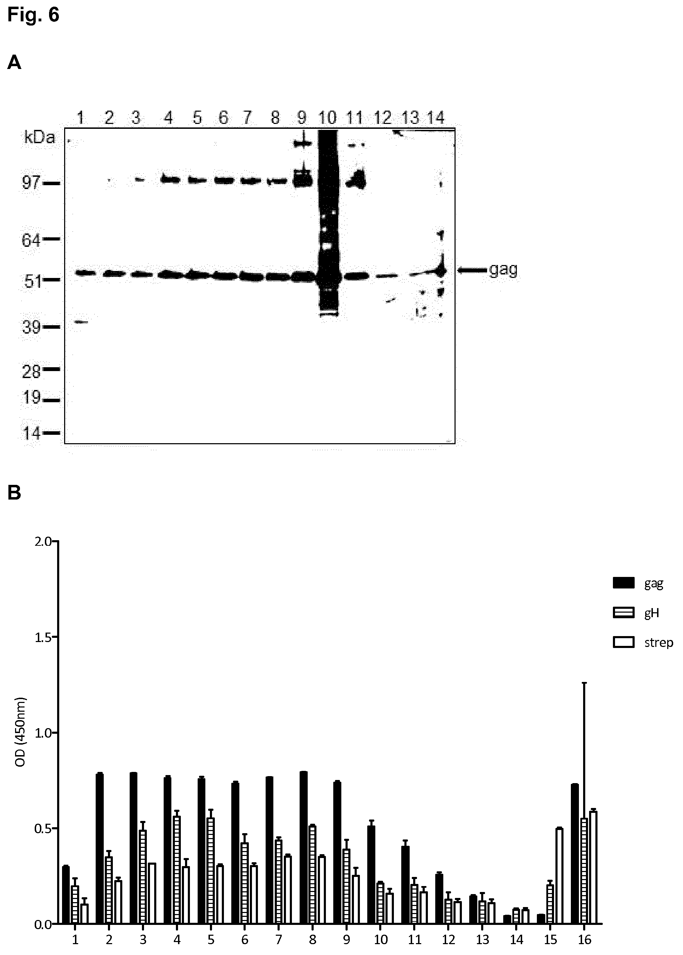

*Figure 6**: Sucrose gradient based purification of non-herpesvirus capsid based CMV-VLP variant SEQ ID NO:20 combined with SEQ ID NO:14.*
   (A) Sucrose gradient based purification. Analysis of different fractions of CMV-VLP variant comprising the capsid (retroviral precursor) protein gag and surface proteins UL75 (gH)-UL115 (gL)-UL128(c)-UL130-UL131A (SEQ ID NO:20 and SEQ ID NO:14) by SDS-PAGE (4-12% Bis-Tris gel) followed by immunoblotting. A mouse anti-gag (DakoCytomation) antibody was used for verification of the capsid protein. Left of the graphic a protein standard in kDa is shown; lanes 1 to 13: fractions 1-13, lane 14: positive control (gag protein).
   (B) Verification of the immunoblot data by ELISA assay using specific antibodies against capsid (gag) and surface (UL75 [gH], streptavidin tagged gL) proteins. Presence of the capsid protein (gag) and surface proteins (gH, gL via streptavidin tag) could be shown by antibody binding to the selected proteins (gag: DakoCytomation; UL75 (gH): Santa Cruz, streptavidin: AbD Serotec). Number 1-13 of the x-axis represents the number of the sucrose gradient fractions 1 to 13 (fractionated from top to bottom), number 14 represents a negative control for VLPs, number 15 a negative control for baculoviruses and number 16 a positive control. Binding strength of antibody to protein in the VLPs is shown as optical density (OD) at the y-axis. Detection of the selected proteins in the same fractions showed their co-localization and therefore intact CMV-VLPs. The same co-localization could be observed by combining the capsid protein gag (SEQ ID NO:20) with the tegument and surface proteins of SEQ ID NO: 9, 10, 13, 15,16 or 19. The inclusion of SEQ ID NO: 19 showed in general higher OD values indicating better stability of the CMV VLP.

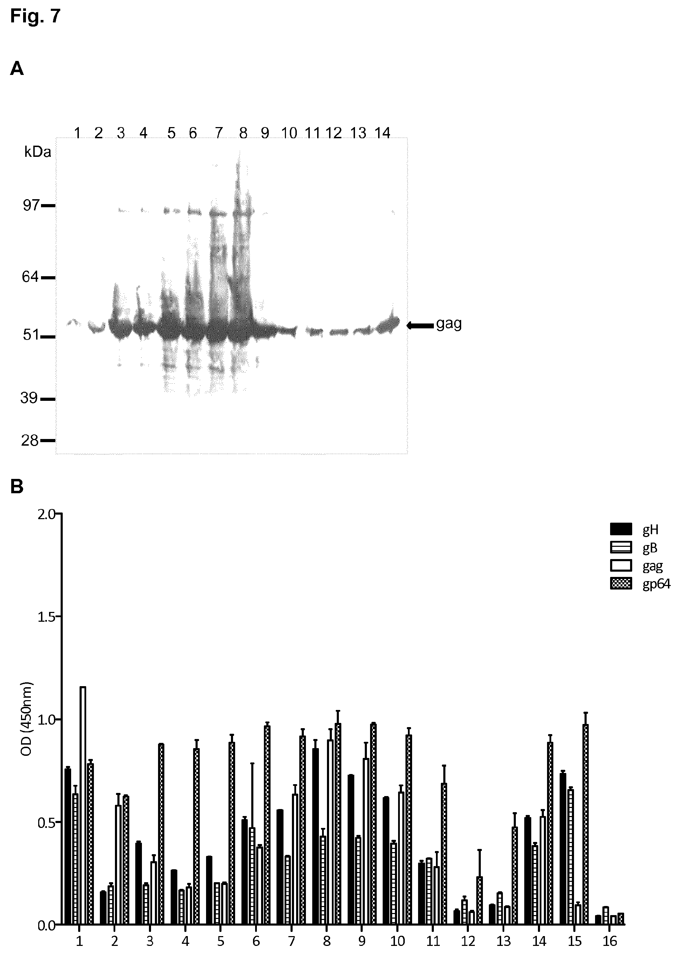

*Figure 7**: Sucrose gradient based purification of non-herpesvirus capsid based CMV-VLP variant SEQ ID NO:20 combined with SEQ ID NO:18.*
   (A) Sucrose gradient based purification. Analysis of different fractions of CMV-VLP variant comprising the capsid (retroviral precursor) protein gag and surface proteins UL75-UL115-UL128-UL130-UL131A-UL55 (SEQ ID NO: 20 and SEQ ID NO:18) by SDS-PAGE (4-12% Bis-Tris gel) followed by immunoblotting. A mouse anti-gag (DakoCytomation) antibody was used for verification of the capsid protein. On the left side a protein standard in kDa is shown; lane 1 to 13 correspond to the fractions 1 to13 from top to bottom fractionation of the gradient and lane 14 represents a positive control.
   (B) Verification of the immunoblot data by ELISA assay using specific antibodies against capsid (gag) and surface proteins (UL75 [gH], UL55 [gB]). Presence of the capsid protein (gag) and surface proteins (UL75 [gH], UL55 [gB]) could be shown by antibody binding to the selected proteins (gag: DakoCytomation; gB: Virusys; gH: Santa Cruz). The presence of baculoviruses was verified with an antibody against the protein gp64 (eBioscience). Number 1-13 of the x-axis represents the number of the sucrose gradient fractions 1 to 13 (fractionated from top to bottom), number 14 represents the load of the gradient and number 15 a positive and number 16 a negative control. Binding strength of antibody to protein in the VLPs is shown as optical density (OD) at the y-axis. Detection of the selected proteins in the same fractions showed their co-localization and therefore intact CMV-VLPs. A further CMV variant based on the expression of SEQ ID NO:22 and SEQ ID NO:18 led to similar results with the benefit to support the induction of cellular immune response by the protein UL83.

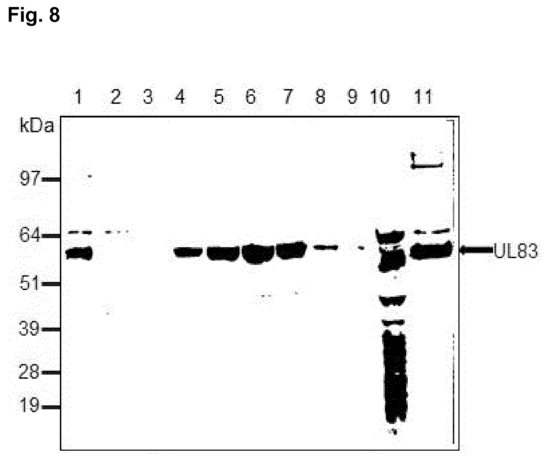

*Figure 8**: Analysis of the His*/*Strep*-*tagged CMV-VLP variant SEQ ID NO:7 combined with SEQ ID NO:14 purified by affinity chromatography.*
   The CMV-VLP variant comprising capsid, functional, tegument and surface proteins UL86-UL85-UL50-UL48A-UL46-UL74-UL83-UL80.5-UL75-UL115(His/Strep)-UL128(c)-UL130-UL131A (SEQ ID NO:7 and SEQ ID NO:14) was purified with an IMAC procedure. Analysis of different steps of this IMAC-purification by SDS-PAGE (4-12% Bis-Tris gel) followed by immunoblotting against the tegument protein (UL83) using a mouse-anti-UL83 antibody (Virusys). On the left side a protein standard in kDa is shown. Lane 1: VLP before purification (load), lane 2: flowthrough (FT), lane 3: wash; lanes 4 to 9: elution with increasing amount of imidazole (55, 100, 150, 230, 480, 500 mM), lane 10: insect cells (Sf9) as negative control, lane 11: positive control (infected Sf9 cells). Presence of the tegument protein indicated by an arrow (UL83).

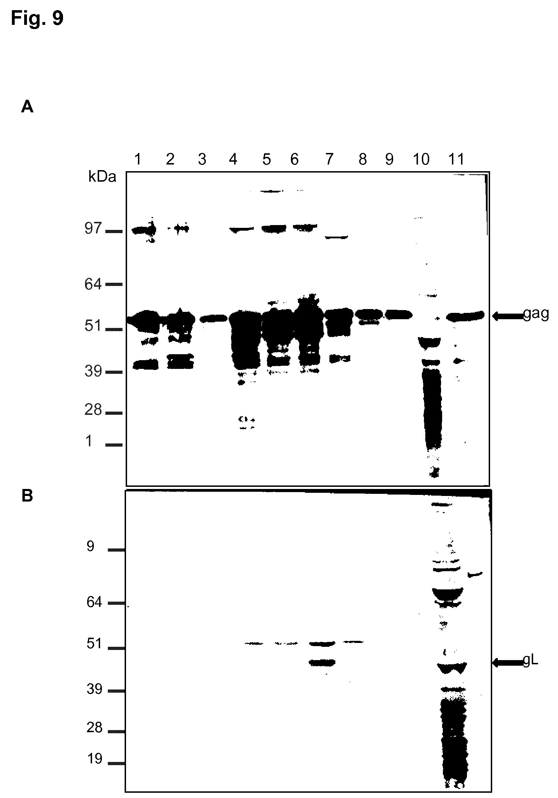

*Figure 9**: Analysis of the His*/*Strep*-*tagged non-herpesvirus capsid based CMV-VLP variant SEQ ID NO:20 combined with SEQ ID NO:14 purified by affinity chromatography.*
   The CMV-VLP variant comprising the capsid (retroviral precursor) protein gag and surface proteins UL75-UL115(His/Strep)-UL128(c)-UL130-UL131A (SEQ ID NO:20 and SEQ ID NO:14) was purified with an IMAC based procedure. Analysis of different steps of this IMAC-purification by SDS-PAGE (4-12% Bis-Tris gel) followed by immunoblotting against the capsid protein (gag, Fig. 9A) using a mouse-anti-gag antibody (DakoCytomation) and against the streptavidin-His tagged surface protein UL115 (gL, Fig. 9B). On the left side a protein standard in kDa is shown, lane 1: VLP before purification (load), lane 2: flowthrough (FT), lane 3: wash; lane 4 to 9: elution with increasing amount of imidazole (55, 100, 150, 230, 480, 500 mM), lane 10: insect cells (Sf9) as negative control, lane 11: positive control (infected Sf9 cells). Presence of the capsid protein (gag) as well as the presence of the streptavidin-histidine tagged surface protein gL indicated by an arrow.

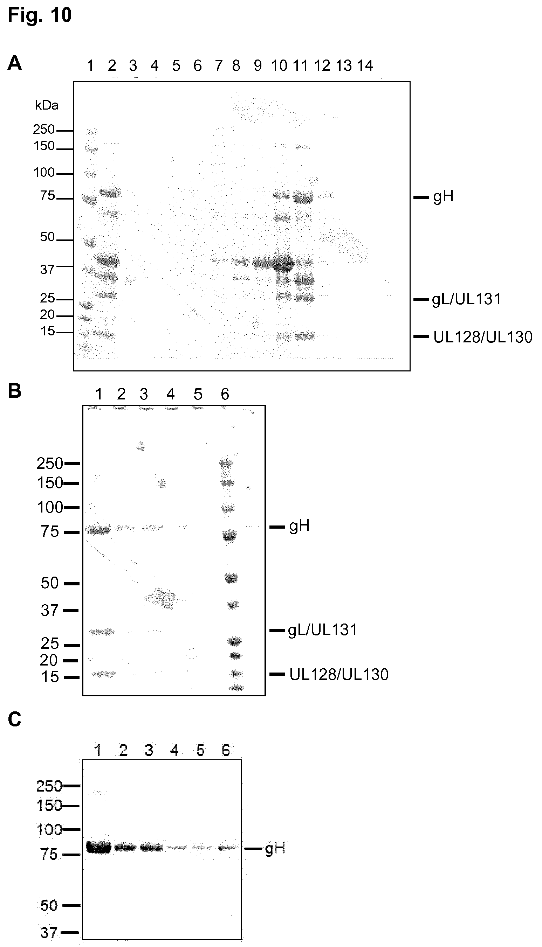

*Figure 10**: Analysis of purification process consisting of two affinity chromatography steps, followed by size exclusion chromatography of the His-tagged soluble CMV-pentameric complex.*
   The pentameric complex comprising the surface proteins UL75 (His-tagged, gH)-UL115 (gL)-UL128-UL130-UL131A (SEQ ID NO: 59) was purified by an affinity based chromatography (IMAC) using His-Trap columns, followed by size exclusion chromatography (XK16/60 Superdex200pg).
   (A) 2^{nd} IMAC purification was analysed by SDS-PAGE (4-12% Bis-Tris gel) followed by coomassie-staining. The sizes (kDa) of the protein standard (lane 1) are marked on the left side. Lane 2: pool of 1^{st} IMAC (load of the 2^{nd} IMAC process), lane 3: flowthrough, lane 4: wash, lanes 5-14 representing elution fractions with increasing imidazole concentration up to 500 mM.
   (B) Analysis of size exclusion chromatography purification by SDS-PAGE (4-12% Bis-Tris gel) followed by coomassie-staining. The sizes (kDa) of the protein standard (lane 1) are marked on the left side. Lane 1: elution pool IV-2, precipitated; lane 2: elution pool IV-2, non precipitated; lane 3: elution pool IV-3; lane 4: elution pool V; lane 5: elution pool VI; lane 6: size marker. Indicated by an arrow are the proteins of the pentameric complex (gH, gL, UL128, UL130, UL131A).
   (C) Immunoblot using antibody against the His-tag of gH. Lane 1 represents the elution pool IV-2 (precipitated); lane 2: elution pool IV-2 (non precipitated); lane 3: elution pool IV-3; lane 4: elution pool V; lane 5: elution pool VI; lane 6: positive control. The His-tagged gH protein is marked on the right side.

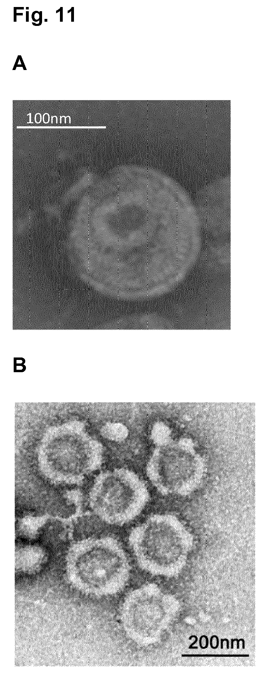

*Figure 11**: Electron micrograph of HCMV*-*VLP variant SEQ ID NO:7 and SEQ ID NO:10 (A) and SEQ ID NO:48 and SEQ ID NO:19 (B).*
   (A) Electron micrograph of recombinant human CMV-VLPs comprising the capsid, functional proteins, tegument proteins and surface proteins UL86-UL85-UL50-UL48A-UL46-UL83-UL80.5-UL74-UL75-UL115-UL128(c)-UL130-UL131A (SEQ ID NO:7 and SEQ ID NO:10 by negative staining with phosphor tungsten acid. The scale bar corresponds to 100 nm.
   (B) Electron micrograph of recombinant human CMV-VLPs comprising the capsid, capsid assembly supporting proteins, tegument proteins and surface proteins UL86-UL85-UL83-UL80.5-UL74-UL75-UL115-UL128-UL130-UL131A-UL55 (SEQ ID NO:48 and SEQ ID NO:19) by negative staining with phosphor tungsten acid. The scale bar corresponds to 200 nm.

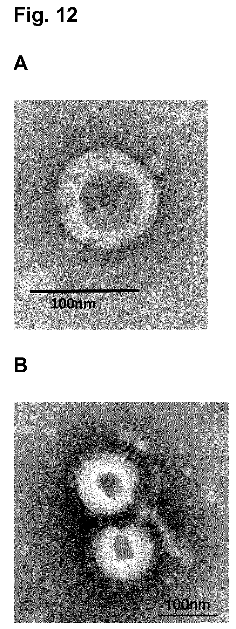

*Figure 12**: Electron micrograph of non-herpes virus based CMV-VLP variant SEQ ID NO:20 and SEQ ID NO:10 (A) and SEQ ID NO:88 and SEQ ID NO:19 (B).*
   (A) Electron micrograph of recombinant human CMV-VLPs comprising the capsid (retroviral precursor) protein gag and surface proteins UL75-UL115-UL128(c)-UL130-UL131A (SEQ ID NO:20 and SEQ ID NO:10) by negative staining with phosphor tungsten acid. The scale bar corresponds to 100 nm.
   (B) Electron micrograph of recombinant human CMV-VLPs comprising the capsid (retroviral precursor) protein MoMULV gag and surface proteins UL75-UL115-UL128-UL130-UL131A-UL55-UL83 (SEQ ID NO:88 and SEQ ID NO:19) by negative staining with phosphor tungsten acid. The scale bar corresponds to 100 nm.

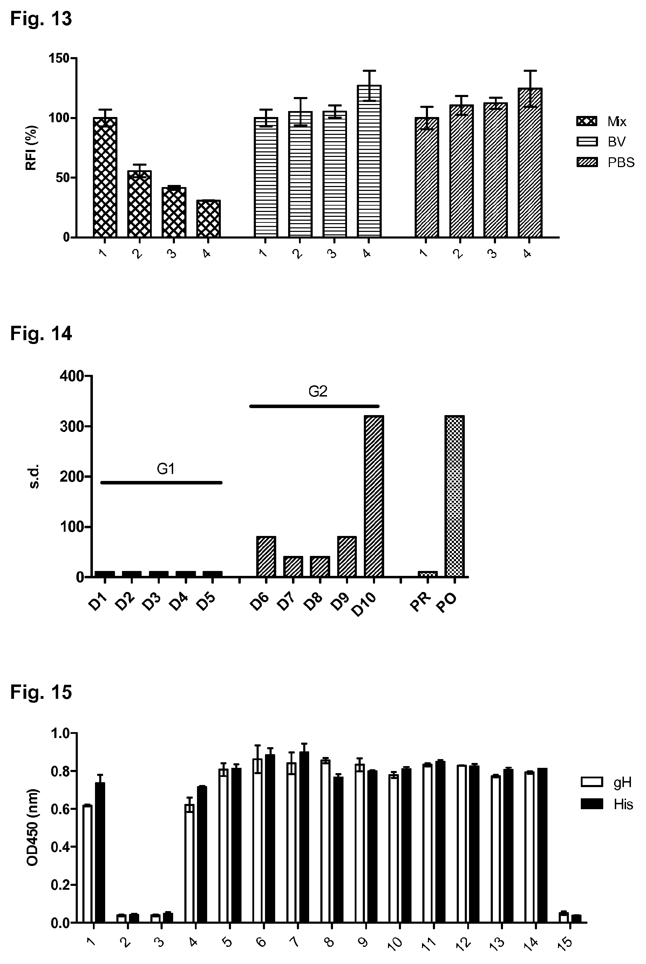

*Figure 13**: Virus neutralization assay with mouse sera based on in vivo studies with a vaccine combination of SEQ ID NO:59 and SEQ ID NO:22 with SEQ ID NO:18, respectively.*
   Balb/C mice were immunized in a prime-boost-boost regimen with hCMV antigens. The selected antigen for this neutralization assay is a protein mix of the soluble complex (SEQ ID NO:59) and the VLP composed of a retroviral capsid in combination with the tegument protein UL83 (SEQ ID NO:22) and the CMV proteins UL55-UL75-UL115-UL128-UL130 and UL131A. Pooled sera obtained from eight mice in a two-fold serial dilution (1:20 to 1:2560) were mixed with a recombinant, BAC-reconstituted VR1814 HCMV strain expressing EGFP under the CMV promoter. MRC-5 fibroblasts (2x10⁴ cells/well) were used to assess the entry of this virus in a cell-based fluorescence assay, 96 h post treatment. The relative fluorescence intensities (RFI, %) are depicted for selected sera dilutions from mice immunized with a mixture of non-herpes CMV-VLP and the pentameric CMV complex. An empty baculovirus and PBS were used as control. The immunisation with a mix of soluble pentameric complex and a non-herpesvirus based CMV VLP induced the generation of neutralising antibodies within a titer range of 1:160 to 1:320 comparable with titers of human blood donors using the pre-evaluated TCID₅₀ for the used EGFP-virus.
   1: w/o serum, 2: 1:320, 3: 1:160, 4: 1:80. Mix: pentamer and VLP; BV: baculovirus.
*Figure 14**: Neutralization assay to verify humoral immune response based on SEQ ID NO:59*
   Comparison of neutralizing antibody titers of human CMV blood donors and mouse sera. Serum from five positive (Group G2, D6-D10) and five negative (Group G1, D1-D5) HCMV blood donors in a two-fold serial dilution (1:20 to 1:2560) were subjected to a cell-based fluorescence neutralization assay. The serum dilution (s.d.) that gave the same inhibitory effect as a 1:320 dilution of pooled sera obtained from eight mice previously immunized with the pentameric complex (SEQ ID NO:59) is shown. The separate analysis of the soluble pentameric complex, as one component in the antigen mix used for immunization of mice, showed the induction of neutralizing antibodies in comparison to human blood donors.
   PR: pentamer pre-immune, PO: pentamer post-immune, G1: negative blood donors, G2: positive blood donors.
*Figure 15**: Quality control of one component (soluble complex, SEQ ID NO: 59) of the vaccine candidate containing the soluble complex and reVLP (recombinant VLP, SEQ ID NO:22 with SEQ ID NO:18).*
   Fractions eluted with different amounts of imidazole are shown after being subjected to an IMAC-based purification. They were tested for the presence of UL75 (gH) and the His tag on gH. The similarity in signal intensity designates the intactness of gH and therefore of the whole complex composed of UL75-UL115-UL128-UL30-UL131A.
   1: load, 2: flowthrough, 3: wash, 4: 250 mM imidazole, 5-8: 300 mM imidazole, 9-13: 350 mM imidazole, 14: positive control, 15: negative control
*Figure 16**: Quality control of one component (soluble complex, SEQ ID NO: 59) of the vaccine candidate containing the soluble complex and the reVLP (recombinant VLP, SEQ ID NO:22*/*18*) *using conformation-dependent antibodies.*
   Different production batches of the complex were tested with a sandwich ELISA assay for the co-presence of the designated proteins. Samples were captured with an anti-gH1 (UL75) antibody and detected with an anti-gH2 and an anti-His as well as anti-UL130/131A and anti-UL130 conformation-dependent (UL130/UL131A) antibodies. The signals confirm co-existence of the proteins in a complex, intactness of the complex, as well as reproducibility of its production.
   1: batch 451-pool 1, 2: batch 459-pool 2, 3: batch 459-pool 1 (15 mM EDTA), 4: batch 459-pool 1 (20 mM EDTA), 5: batch 458 (15 mM EDTA), 6: positive control, 7: negative control, 8: internal standard
*Figure 17**: Validation of a non-herpes-based CMV-VLP (SEQ ID NO: 22 combined with SEQ ID NO: 18) with sandwich ELISA* assay.
   Different elution fractions were tested for the co-presence of the designated proteins. Samples were captured with a conformation dependent antibody (UL130/UL131A) giving an indication that the surface proteins UL130 and UL131A are presented in the natural conformation. An anti-gH (UL75), an anti-gpUL83 (pp65) and an anti-capsid (gag) antibody were used for detection. The signals confirm co-existence of proteins on a VLP, and reveal the enrichment of some samples in the proteins tested.
   1: bulk, 2: fraction 7, 3: fraction 8, 4: fraction 9, 5: fractions 8+9, 6: negative control. nOD: normalized OD, cap: capsid.
*Figure 18**: Characterization of a purification process of a non-herpes-based CMV-VLP (SEQ ID NO: 22 combined with SEQ ID NO: 18) with a sandwich ELISA* assay.
   Elution fractions from two different chromatography matrices (monolith: c1 and membrane adsorbers: c2) were tested for the co-presence of the designated proteins. Samples were captured with a conformation-dependent (UL130/UL131A) antibody and detected with an anti-gH, an anti-pp65, an anti-gB and an anti-gag (capsid) antibody. The signals confirm co-existence of the proteins on a VLP, and reveal the better performance of the membrane adsorber (c2) used.
   1: elution fraction from column 1 (c1), 2: elution fraction from column 2 (c2), 3: positive control, 4: negative control. Cap: capsid (precursor gag protein).

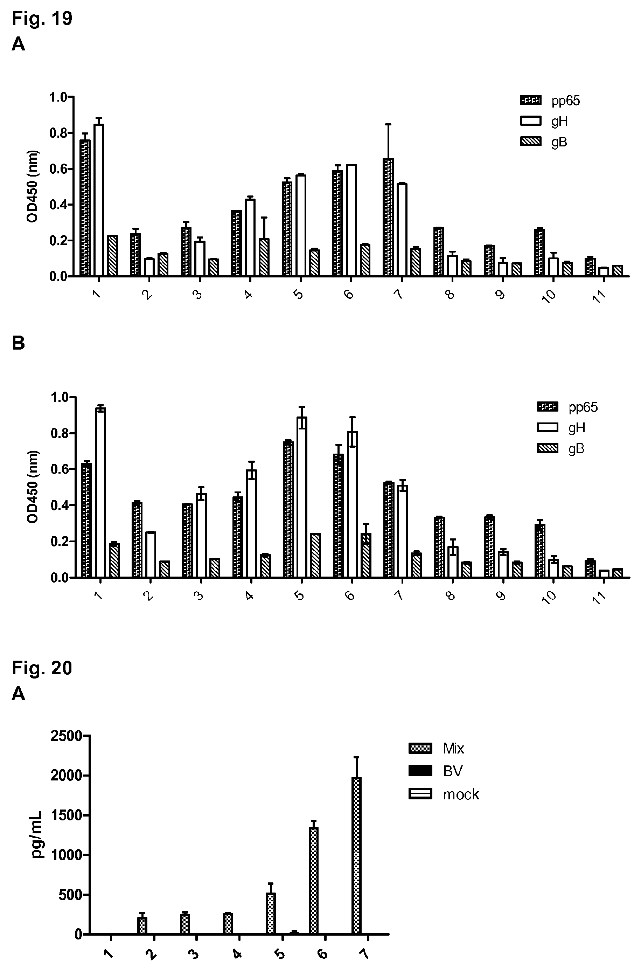

*Figure 19**: Characterization of a herpes-based CMV-VLP with sandwich ELISA assay.*
   VLPs were produced with two (Figure 19A, UL86 and UL80.5, SEQ ID NO:2) or three (Figure 19B, UL86-UL85-UL80.5, SEQ ID NO:3) different capsid proteins; the same tegument protein UL83 (pp65) and different surface proteins (gH-gL-gB-UL128-UL130-UL131A, SEQ ID NO:18), and elution fractions from the subsequent purification step were subjected to sandwich ELISA. Samples were captured with a conformation- dependent (UL130/UL131A) antibody against surface proteins and detected with antibodies against tegument and surface proteins (anti-pp65, anti-gH and anti-gB). Both capsid types led to VLPs, yet with different yield. Integration of the gB protein appeared to be beneficial for the core consisting of more than three proteins. 1: bulk, 2-10: fractions 4-12, 11: negative control.

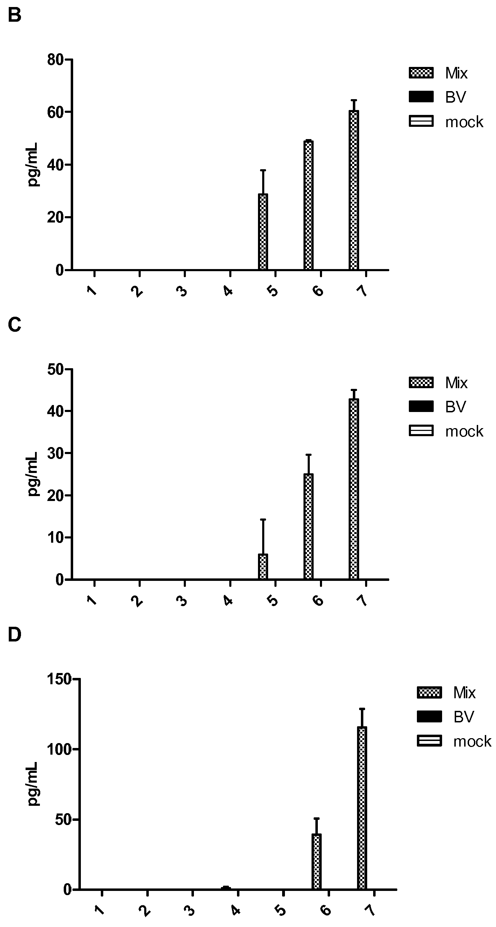

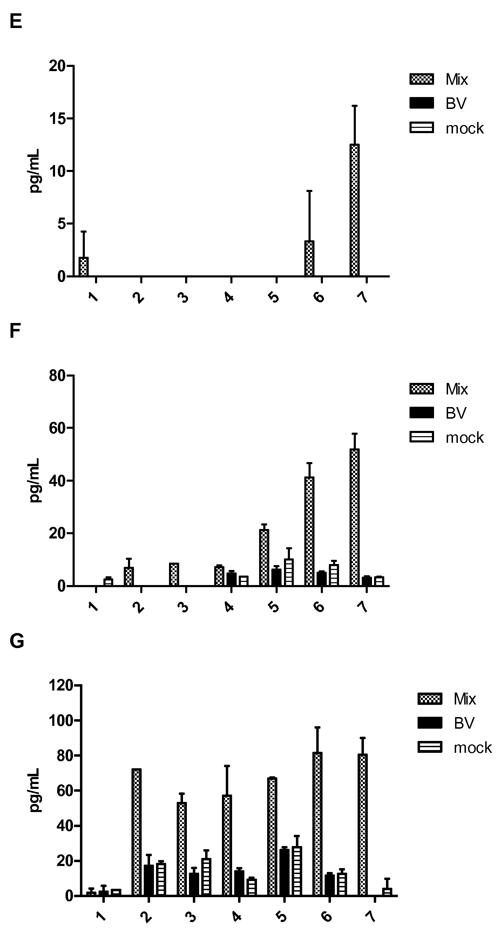

*Figure 20**: Cellular immune response induced by a combination of a non-herpes CMV-VLP (SEQ ID NO:22*/*SEQ ID NO:18) and the pentameric CMV complex (SEQ ID NO:59).*
   Spleen cells were re-stimulated 24 h post harvesting with various peptides (1-7), and the release of the Th1-type cytokines, IFN-g (A) and IL-2 (B), the Th2-type cytokines, IL-4 (C), IL-5 (D) and IL-10 (E), and the inflammatory cytokines, GM-CSF (F) and TNFa (G) was measured with a multiplex assay according to manufacturer's protocol (Invitrogen). Peptides 2-5 were a mixture of nonamers with predicted epitopes (SYFPEITHI program), whereas peptides 6 and 7 were commercially purchased (JPT, mix of pre-defined peptides). An empty baculovirus and PBS (mock) were used as controls for the restimulation.
   1: mock, 2: gH, 3: gB, 4: UL128/130, 5: UL131/gL, 6: pp65, 7: Gag capsid. Mix: pentamer and VLP; BV: baculovirus.

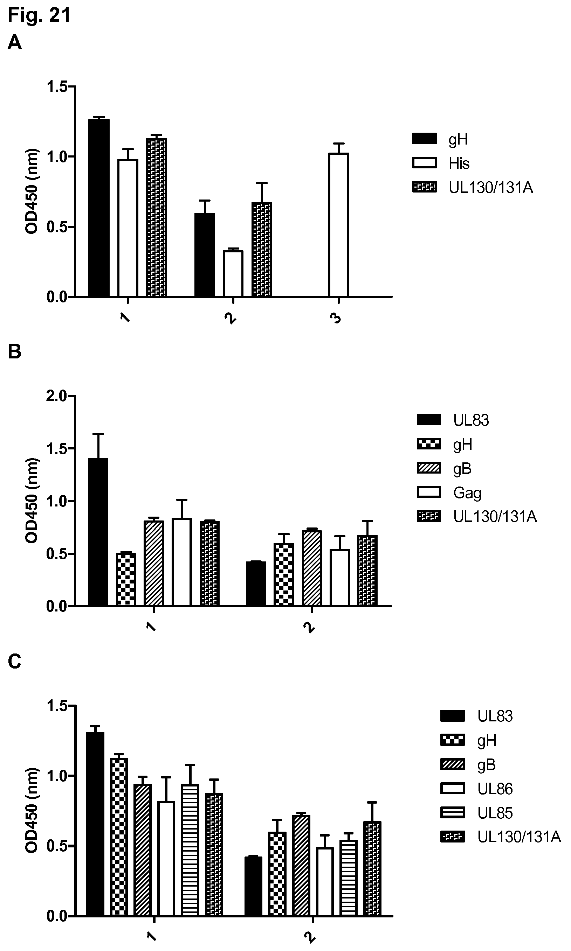

*Figure 21**: Analysis of CMV VLPs and single components (soluble complex, SEQ ID NO:59) for a vaccine using human CMV positive sera.*
   Non-herpes virus based CMV VLP (SEQ ID NO:22 combined with SEQ ID NO:19), herpes virus based CMV VLPs (SEQ ID NO:48 combined with SEQ ID NO:19) and the soluble complex (SEQ ID NO:59) were tested in a sandwich ELISA assay for their ability to react with human antibodies from CMV-positive donors to simulate an *in vivo* experiment. Plates were coated with antibodies corresponding to different CMV proteins (gH, gB, UL83, UL85, UL86 and conformation-based UL130/UL131A), to Gag and to His. The plates were further incubated with either the soluble pentameric complex or VLPs and subsequently treated with CMV-positive human sera (HIV negative donors). An HRP-labeled secondary human anti-IgG was used for detection.
   (A) The pentameric complex gH-gL-UL128-UL130-UL131A (SEQ-ID NO:59) was analyzed with human sera in a sandwich ELISA assay. gH and UL130/UL131A in the complex are recognized by human antibodies in donors' sera (mix of 4 different sera). 1:pentameric complex; 2: negative control (mock); (3) positive control (His-expressing protein).
   (B) A gag-based CMV VLP gag-UL83-gL-gH-UL128-UL130-UL131A-gB-UL83 (SEQ-ID NO: 22 combined with SEO-ID NO:19) was analyzed with human sera in a sandwich ELISA assay. UL83 in these VLPs is recognized by human antibodies in donors' sera. 1: Gag-based CMV VLP; 2: negative control (mock). Very similar results were obtained for a CMV VLP based on the MoMULV gag capsid (SEQ-ID NO: 88 combined with SEQ ID NO:19)
   (C) A CMV-based VLP UL86-UL85-UL83-UL80.5-UL74-gL-gH-UL128-UL130-UL131A-gB-UL83 (SEQ-ID NO: 48 combined with SEQ-ID NO:19) was analyzed with human sera in a sandwich ELISA assay. All proteins in these VLPs were recognized by human antibodies in donors' sera with very clear signals for UL83, gH, UL85, UL86. 1: Gag-based CMV VLP; 2: negative control (mock). The remaining baculovirus in the CMV-VLP and soluble complex manufacturing did not bind to the antibodies in present in human sera.

### Detailed Description of the Invention

The invention relates to novel virus-like particles for use as anti-HCMV vaccine, for development of therapeutic antibodies, in anti-HCMV treatment, as diagnostic tools and as R&D tools. The present invention describes several possibilities to generate recombinant, non-infectious CMV-particles leading to a humoral and cellular immune response. Various protein combinations are generated in order to obtain a product with the desired properties. More specifically the present invention focuses on multicomponent VLP variants and combinations of different protein compositions such as VLPs, protein complexes, soluble proteins and/or DNA based compositions such as vectors and peptides.

The recombinant VLPs of the present invention are based on a capsid formed by either a CMV capsid protein combination of 1 to 6 proteins (major capsid, minor capsid, smallest capsid protein and necessary proteins for assembly) or on a retroviral precursor protein, in particular the one of the lentiviral human immunodeficiency virus (HIV) named gag, and/or pr55. Further proteins are added to these capsid proteins selected from CMV tegument proteins and surface proteins. The VLP formed is surrounded by an envelope provided by the host cell during the secretion process. In order to connect and/or embed the tegument and surface proteins in their natural conformation, their individual membrane anchors are present.

Since a CMV virus contains at least 5 capsid proteins (gene products of UL46, UL48A, UL85, UL86, UL104), 19 regulatory proteins, 17 tegument proteins (gene products of UL25, UL45, UL47, UL48, UL69, UL71, UL72, UL76, UL77, UL83, UL88, UL93, UL94, UL95, UL97, UL99, UL103), 5 surface or envelope proteins (gene products of UL55 [gB], UL73 [gN], U74 [gO], UL75 [gH], UL100 [gM], UL115 [gL]), the non-categorized gene products from the open reading frame UL128, UL130, UL131A; proteins from 15 beta-herpesvirus specific genes (UL23, UL24, UL32, UL33, UL35, UL36, UL38, UL43, UL74 [gO], UL78, UL82, UL96, IRS1, US22, TRS1) and so-called functional proteins from the open reading frames (ORF) UL50, UL80.5 known to date, theoretically up to 1x10⁶⁴ combinations would be possible and the largest VLP could contain more than 40 proteins, whereby the regulatory proteins are not taken into account. So far no recombinant CMV VLP exists due to the complexity of this virus.

In the present invention, the term "surface protein" is used, but is equivalent to the term "envelope protein".

The invention relates to a recombinant virus-like particle comprising one or more capsid or capsid precursor proteins, 3 or more different surface proteins from cytomegalovirus (CMV), and optionally one or more tegument proteins.

In a preferred embodiment of the invention the surface proteins are selected from the group consisting of pUL83 (pp65), gpUL75 (gH), gpUL55 (gB), gpUL100 (gM), gpUL73 (gN), gpUL74 (gO), gpUL115 (gL), gpUL75 (gH), gpUL128, gpUL130, and gpUL131A, preferably from human CMV. Nevertheless proteins of non-human CMV strains may be used. Such non-human CMV proteins then may take a structural rather than an immunological function in the VLP. The recombinant VLPs according to the invention may comprise proteins from other herpesvirus families such as HSV (herpes simplex virus), EBV (Epstein-Barr virus) and KSHV (Kaposi's sarcoma-associated herpesvirus).

To address the species specificity, the different cell tropism and the induction of a broad immune response the VLPs of this invention contain more than one surface protein in combination with a number of capsid and tegument proteins.

In another preferred embodiment, the VLPs based on CMV capsid proteins are composed of one protein or of combinations of proteins selected from the six proteins pUL86, pUL85, pUL48A, pUL46, pUL50, and pUL80.5, for example two, three, four, five, or six of these proteins. In a further aspect of the invention the VLP comprises protein pUL104.

The non-herpesvirus capsid is chosen from the *retroviridae* out of the retroviral families, alpha-, beta-, gamma- , delta and epsilon retrovirus, the lentivirus family or the spumavirus family. Out of the lentiviral family the avian (SIV), feline (FIV), bovine (BIV) and the human (HIV) precursor protein gag (group-specific-antigens) is used as capsid for the generation of non-herpesvirus based VLPs as well as the precursor protein gag from the gamma retrovirus, like feline leukemia virus (FELV), moloney mouse leukemia virus (Mo-MuLV), avian leukemia virus (SLV) or the murine sarcoma virus (MSV). The VLPs based on a non-herpesvirus capsid are preferably composed of the HIV precursor protein gag (pr55), which is a precursor protein composed of a matrix (MA, p17), a capsid (CA, p24), a nucleocapsid (NC, p7) and a link (LI, p6) protein part.

It is known that there are no huge differences in the assembly of the gag precursor proteins from different retroviral family members. Besides the HIV gag the MoMULV gag variant, which is composed of the same subunits, is suitable as retroviral based capsid carrying more than three different CMV tegument and/or surface proteins for the formation of a CMV VLP, as well as having advantages in the manufacturing and regulatory approval of a vaccine. The assembly of CMV VLP variants based on the MoMuLV gag precursor protein is similar to the assembly of HIV gag based VLPs. There is no big advantage in respect to yield, better shape and conformation of the MoMULV gag based CMV-VLPs. However, in contrast to a HIV based CMV VLP human vaccine no false positive results in a diagnosis test for HIV are expected for a CMV-VLP vaccine based on a MoMuLV gag capsid.

A preferred VLP comprises, on top of the respective capsid proteins, one or two tegument proteins selected from the group consisting of UL83, UL25, UL32 (pp150), and UL99, and five to eight surface proteins. The surface proteins are preferably selected from the group consisting of UL75 (gH), UL115, (gL), UL74 (gO), UL55 (gB), UL128, UL130, and UL131AA. In a further embodiment of the invention the VLP may also contain UL100 (gM) and UL73 (gN). The surface proteins of the HCMV in the VLPs of the invention are selected together with their respective membrane anchor such that they are displayed and embedded in their natural conformation into the recombinant capsid structure.

In one embodiment the CMV capsid contains only the major capsid protein (UL86). In all other embodiments the capsid consists of more than one protein. In the preferred embodiment the VLP contains pUL86-pUL85-pUL80.5 in combination with tegument proteins pUL83 and ppUL32 (pp150) and the surface proteins gpUL75 (gH), gpUL115 (gL), gpUL55 (gB), gpUL128, gpUL130, and gpUL131A. With the same preference the mentioned tegument and surface proteins are expressed on a capsid composed of pUL86-pUL85-pUL48A improving the core and therefore the VLP shape.

In another preferred embodiment the VLP additionally comprises surface protein gpUL74 (gO). This enhances particle formation and enlarges the amount of proteins inducing protective immune response.

In a further aspect of this invention the capsid and tegument proteins are from another herpesvirus, such as herpes simplex virus, Epstein-Barr virus, HHV-6 (roseolovirus) or HHV-7 (pityriasis rosea).

In a further aspect of this invention the VLPs comprise at least one tegument protein and/or one surface protein of non-human CMV origin.

In another embodiment of this invention the VLPs comprise at least two different proteins fused to a peptide comprising a coiled-coiled motif, having a zipper function and connecting said at least two proteins carrying the corresponding motif. Preferred proteins fused to the peptide comprising a coiled-coiled motif are one of the capsid proteins, preferably UL86, and one of the surface proteins, preferably to gpUL75, to support the assembly into a functional VLP and to increase the amount of immunogenic proteins on the surface.

In one embodiment of the present invention the proteins for the generation of a multicomponent recombinant VLP are chosen from a single CMV strain, preferably selected from Towne, Toledo, AD169, Merlin, VR1814 strain, and/or clinical isolates. In another embodiment the proteins for the generation of the VLPs are selected from different CMV strains, preferably from Towne, Toledo, AD169, Merlin, VR1814 strains and/or different clinical isolates.

VLPs comprising protein sequences from different CMV strains confer protection against several strains and prevent re-infection and/or re-activation with similar strains. Different CMV strains considered are Towne, Toledo, AD169, Merlin, TB20, and VR1814 strains, but also other clinical isolates.

These strains are considered for several reasons, such as existing data of clinical phase II studies with vaccine candidates using Towne and Toledo strains, well characterized AD169 based data and commercially available tools for analysis of the manufacturing process. Furthermore the clinical isolate VR1814 contains functional complexes which are important for entry into different cell types such as fibroblasts and epithelial/endothelial cells. For the bacterial artificial chromosome (BAC) derived and laboratory adapted Towne strain it is known that the entry into epithelial cells is blocked because of non-functional protein compositions on the surface. By combining different strains in the recombinant CMV VLPs the natural malfunctions are circumvented. Merlin and TB20 are now more often used as reference strains. New technologies such as next-generation sequencing allow the determination of immunogenic epitopes from CMV positive donors. The genetic information of the corresponding virus (clinical isolate) is used to generate an improved vaccine. Therefore the CMV VLPs are composed of proteins from different virus strains.

Preferred are Towne and VR1814 combinations of protein sequences, in particular in a VLP composed of gpUL55, gpUL74, gpUL75, gpUL115, UL128, UL130, and UL131A, and either CMV capsid protein(s) or retroviral gag capsid protein.

The protein compositions of the invention contain immunogenic proteins and therefore a number of different epitopes, preferably of the kind that stimulate humoral and cellular immune responses. The activation of humoral immune responses by epitopes leads to the production of antibodies that will bind to proteins containing such epitopes. The activation of cellular immune responses by epitopes leads to the production of cytotoxic T cells (also known as T_{C}, CTL, T-Killer cell, cytolytic T cell, CD8+ T-cell, or killer T cell) which kill cells presenting such epitopes on their surface. Epitopes as understood herein may be repetitive, and may be part of a larger protein, in particular part of an antigen.

Different proteins comprising epitopes of the invention are, for example, gpUL75 (gH), gpUL115 (gL), UL128, UL130, UL131A, gpUL55 (gB), gpUL74 (gO), gpUL100 (gM), gpUL73 (gN), pUL86, pUL85, pUL80.5, pUL82, pUL83, pUL46, pUL48A, pUL50, pUL32, and the immediate early protein IE-1.

In a preferred embodiment the VLPs comprise epitopes from a single or from different HCMV strains and/or non-human CMV strains, combined with B- and/or T-cell epitopes in order to induce a broader immune response.

VLPs comprising an inclusion of CD ligands (e.g. CD40L, CD19L) are another aspect of the present invention. Such VLPs induce the immune response *via* a distinct pathway resulting in a Th1 or Th2 answer.

A further aspect of the present invention are VLPs comprising CpG motives, cytokines, and/or betaglucans, Such VLPs directly trigger B-cell activation, and enhance and broaden the immune response caused by the specific HCMV epitopes.

In another preferred embodiment the virus-like particle consists of proteins forming a complete virus-like surface, optionally further comprising a viral capsid protein. The virus-like particle of the invention may further comprise fluorescent proteins, proteins useful for purification purposes of the particles or for attaching a label, and proteinaceous structures required for transport processes.

The invention relates to a recombinant virus-like particle comprising multiple combinations of capsid, optionally tegument and/or surface proteins from non-human and/or human cytomegalovirus in different amounts. These particles contain at least a capsid protein, preferably the major capsid protein UL86, a tegument protein selected from the group of ppUL 83, ppUL32, pUL25, and the IE-1 protein, and a surface protein selected from the group consisting of gpUL75 (gH), gpUL115 (gL), gpUL128, gpUL130, gpUL131A, gpUL55 (gB), gpUL74 (gO), gpUL100 (gM), and gpUL73 (gN). The surface proteins are preferably of human viral origin. The virus-like particles of this invention consist either of one or more, such as one to sixteen proteins comprising various proteins selected either (a) from one HCMV strain or (b) from different HCMV strains and/or (c) from non-human CMV like mouse or guinea pig CMV. Preferred are recombinant virus-like particles comprising one or more, preferably two or more different proteins from a single HCMV strain or different proteins from different CMV strains. Likewise preferred are recombinant virus-like particles comprising one or more capsid proteins, the tegument proteins pUL83 and ppUL32, and the surface proteins gpUL55 (gB), gpUL74 (gO) and/or the proteins forming a pentameric structure selected from the group consisting of gpUL75 (gH), gpUL115 (gL), gpUL128, gpUL130, and gpUL131A. The recombinant virus-like particles of this invention may also be based on a non-herpesvirus capsid, preferably on the lentiviral precursor protein gag, and further be composed of CMV surface proteins and optionally CMV tegument proteins.

In preferred embodiments a virus-like particle of the invention comprises:
a. one capsid protein from a single CMV strain or a non-herpesvirus strain;
b. a mix of two or more capsid proteins from a single CMV strain;
c. a mix of two or more capsid proteins from different CMV strains;
d. a combination of one capsid, one tegument and one surface protein from a single CMV strain;
e. a combination of one capsid, one tegument and one surface protein from different CMV strains;
f. a combination of one or more capsid proteins; one or more tegument proteins and one or more surface proteins from a single CMV strain;
g. a combination of one or more capsid proteins; one or more tegument proteins and one or more surface proteins from different CMV strains;
h. a combination of one or more capsid proteins, one or more tegument proteins, and one or more surface proteins from one or more CMV strains with additional T-cell epitopes;
i. a combination of one or more capsid proteins, one or more tegument proteins, and one or more surface proteins from one or more CMV strains excluding proteins responsible for immune evasion;
j. a combination of one or more capsid proteins, one or more tegument proteins, and one or more surface proteins from one or more CMV strains excluding proteins responsible for inhibition of MHCI and MHCII expression; and
k. a combination of one or more capsid proteins, one or more tegument proteins, and one or more surface proteins from one or more CMV strains further comprising proteins and/or motives enhancing the immune response, selected from the group of CD40L, CD19L, cytokines and unmethylated CpG motives;
l. a combination of one or more capsid proteins, one or more tegument proteins, and one or more surface proteins from one or more CMV strains with proteins facilitating purification and analysis, selected e.g. from the group consisting of His-tag, Strep-tag, myc-tag, Flag-tag, and Snap-tag, and fluorescence proteins, selected e.g. from the group consisting of GFP, YFP and Red-cherry;
m. a combination of one or more capsid proteins, one or more tegument proteins, and one or more surface proteins from one or more CMV strains comprising further proteinaceous or non-protein structures to couple chemicals and/or inert nanobeads.

The strategy presented in the current invention to obtain large and complex CMV VLPs includes co-expression of multiple genes from single vectors, including genes for CMV capsid, tegument and/or surface proteins, as well as co-infection of several of these co-expression vectors.

The production of these novel multicomponent virus-like particles, protein complexes and soluble proteins is based on the recombinant assembly technology (known as rePAX) which includes the following steps: the assembly of genes, the generation of expression constructs for various systems, the protein expression, purification and characterization of the product. The preferred expression system for the platform is the baculovirus system (BEVS) or the mammalian cell system. In another embodiment of the present invention a combination of both systems, known as BacMam, is used.

The herein described protein compositions such as VLPs, protein complexes and single proteins are generated in a shorter time and in unlimited amounts, due to the use of specific genetic and process engineering tools. The capability to assemble the required genes by modern molecular biology methods, such as the described rePAX technology and gene synthesis, for instance, allows fast assembly of the coding DNA vector. The use of these technologies does not require any physical transfer of original, potentially dangerous infectious or carcinogenic material during the development, manufacturing or administration of virus-like particles, protein complexes, and soluble proteins. Thus the vaccine of the invention is safe. For the construction of protein compositions of the invention it is sufficient to use nucleotide sequences from HCMV available at the NCBI database.

The DNA encoding the proteins forming the VLPs of the invention, and vectors, either viral or plasmid based, comprising such DNA, are also part of the invention.

For the generation of expression constructs for the BEVS system the vector backbone pRBT136, containing elements for propagation in *E.coli* and yeast (e.g. *Saccharomyces cerevisae*.) and for protein expression in insect cells are preferably used.

For expression in the mammalian system the vector backbone pRBT393 containing elements for gene assembly, homologous recombination and for expression in mammalian cells, preferably HEK293, CHO, human foreskin fibroblasts (HFF) and epithelial cells, are preferably used.

The parallel assembly of the different genes is conducted by homologous recombination of PCR products with homologous flanking regions and containing expression cassettes (promoter - gene of interest - terminator) or single genes fused together with protease cleavage sites such as the foot-and-mouse-disease virus 2A cleavage site into one open reading frame (ORF). The promoters are preferably selected from the group consisting of polh, p10 and p_{XIV} very late baculoviral promoters, vp39 baculoviral late promoter, vp39polh baculoviral late/very late hybrid promoter, pca/polh, *pcna, etl, p35, egt,* da26 baculoviral early promoters; CMV-IE1, UBc. EF-1, RSVLTR, MT, p_{DS47}, Ac5, and P_{GAL} and P_{ADH}. The terminators are preferably selected from the group consisting of SV40, HSVtk and BGH (bovine growth hormone).

The vector backbone pRBT136 used preferably for this invention contains an origin of replication for *E.coli,* e.g. pBR322ori, and yeast, e.g.2 micron ori, the polh and p10 promoters for expression in insect cells, the terminators SV40 and HSVtk, several resistance markers (ampicillin, gentamycin), a yeast selection marker (URA3), transposon sites (Tn) and a multiple cloning site (MCS).

An expression cassette containing promoter - gene of interest - terminator is PCR amplified at the 5' site with a 35-40nt overhang at the 5' site, and at the 3' site with a further and different 35-40nt overhang. For homologous recombination with a second expression cassette, having the same organization, the PCR product contains, at the 5' site, the complementary sequence of the 35-40nt overhang to the 3' site of the previous PCR product. The remaining overhangs at the 5' site of the first PCR product and the 3' site of the second PCR product are homologous to the 3' and the 5' end of a linearized vector (pRBT136), respectively. The homologous recombinations in a sequence are then conducted in yeast, preferably in *Saccharomyces cerevisiae.* The number of the expression cassettes/PCR products to be assembled in parallel with the strategy described before is increased according to the needed number of genes to be assembled. By this means multiple genes / expression cassettes are assembled in parallel. The assembled genes are flanked by the transposon sites. These are used for transposition of the genes into the baculovirus genome. The resulting baculovirus co-expression vector ensures that the genes are co-expressed from the same single cell. Yield and product composition vary dependent on the number of proteins and production parameters. The production parameters such as cell line, cell count at infection (CCI), amount of recombinant virus inoculum (multiplicity of infection, MOI) and time of harvest (TOH) are determined in respect to yield and early harvest using a matrix system and a small scale production system (2-20 ml; Ries, C., John C., Eibl R. (2011), A new scale down approach for the rapid development of Sf21/BEVS based processes - a case study. In Eibl R., Eibl D. (Editors): Single-use technology in Biopharmaceutical Manufacture, 207-213, John Wiley & Sons, Hoboken, New Jersey). The defined parameters are then used to produce the respective product at larger scale. Particles of the invention are manufactured using modern disposable tissue culture techniques which allow for high production capacity.

The preferred production cell lines of this invention are insect cell lines such as Sf9, Sf21, Hi-5, Vankyrin (VE-1, VE-2, VE-3), Express Sf+, and S2 Schneider cells. For expression in mammalian cells, in particular human cells, e.g. HEK293, CHO, HeLa, Huh7, HepG2, BHK, MT-2, human foreskin fibroblasts (HFF), bone-marrow fibroblasts, primary neural cells, or epithelial cells are used. For expression in yeast S. *cerevisiae, S. pombe, C. albicans,* or *P. pastoris* cells are used.

Cultivation and propagation of host cells according to the invention is done in any vessel, bioreactor or disposable unit providing the appropriate conditions for the particular host cell.

The herein described CMV particles are purified using ultracentrifugation based classical glycerol-tartrate and sucrose gradients or modern chromatography based techniques, preferably affinity (IMAC) and ion exchange chromatography using large porous matrices such as membrane adsorbers and monoliths.

The virus-like particles of the invention are used as therapeutics and/or as prophylactic vaccines. Furthermore they are used as antigens in diagnostic tools and as antigens for antibody generation.

For both types of vaccines, either prophylactic or therapeutic, the antigen specific CD8+, cytotoxic T cell response as well as the antigen specific CD4+, T helper cell response is most important. Therefore the protein compositions of the VLPs are adapted to induce T-cell by expressing, e.g., pUL83, a known T-cell stimulator. In order to further address the different requirements of preventive vaccines, the present invention contains combinations of VLPs, soluble protein complex and single proteins. The compositions of the VLPs are adapted to contain immunogenic glycoproteins (gpUL75, gpUL55) and proteins necessary for formation of distinct complexes (gpUL115, gpUL128, gpUL130, gpUL131A). These combinations comprise the combination of soluble pentameric complex [gpUL75 (gH), gpUL115 (gL), gpUL128, gpUL130, gpUL131A] and virus-like particles, combination of one or more single soluble protein (such as pUL83, gpUL55) and the virus-like particles, and combination of the virus-like particles, the pentameric complex and a single soluble protein. The combinations are aimed at enhancing the immune response in general and induce generation of neutralizing antibodies and a long-lasting response. For the induction of neutralizing antibodies the glycoproteins gpUL75 (gH), gpUL115 (gL) and gpUL55 (gB) are essential targets and are therefore expressed on the surface of the CMV-VLPs, preferably of the HCMV-VLPs.

A further aspect of this invention is the use of CMV-VLP and soluble pentameric complex (gpUL75 (gH), gpUL115 (gL), gpUL128, gpUL130, gpUL131A) in a prime-boost regimen, preferably priming with the VLPs and boosting with the pentameric complex. Priming with the pentameric complex and VLP boost is a suitable vaccination scheme. The combination of VLP and soluble protein and/or VLP and DNA in such a prime-boost scenario is another aspect of this invention.

In a further aspect the invention relates to a vaccine comprising a recombinant virus-like particle as described, in particular to such a vaccine further comprising the pentameric complex consisting of gpUL75, gpUL115, gpUL128, gpUL130 and gpUL131A, and/or soluble CMV protein selected from the group consisting of gpUL75, gpUL115, gpUL55, gpUL74, gpUL100, and gpUL73; or gpUL128, gpUL130, and gpUL131A; or pUL83, IE-1, UL99, UL91, and pp150. Such a vaccine may further comprise an adjuvant selected from the group consisting of aluminium hydroxide, alum, AS01, AS02, AS03, AS04, MF59, MPL, QS21, ISCOMs, IC31, unmethylated CpG, ADVAX, RNA containing formulations and Freund's reagent, but also DNA of the invention as defined above.

To reduce the chance of congenital disease a prophylactic vaccine to prevent the first CMV infection of the mother is desirable, whereas an effective therapy is needed in the case a mother is diagnosed with an active CMV infection.

The pharmaceutical compositions of the VLPs of this invention are used in a method of prophylaxis. A method for vaccinating a human uses a pharmaceutical composition of the present invention comprising VLPs in the range of 10 µg to 10 mg/dose. An average human of 70 kg is assumed to receive at least a single vaccination. Preferably a dosage regimen comprising 3 doses applied at 0, 8 and 24 weeks, optionally followed by a second vaccination round 12-24 months after the last immunization is chosen. Preferred routes of administration are subcutaneous (sc) and intramuscular (im) administration, but intradermal and intranasal are also suitable administrations.

The pharmaceutical compositions of the VLPs of this invention are likewise used in a method of therapeutic treatment. A method for vaccinating a human for treatment purposes uses a pharmaceutical composition of the present invention comprising VLPs in the range of 10 µg to 10 mg/dose. An average human of 70 kg is assumed to receive at least a single vaccination. Preferably a dosage regimen comprising 3 doses applied at 0, 8 and 24 weeks, optionally followed by a second vaccination round 12-24 months after the last immunization is chosen. The pharmaceutical composition of the present invention comprises VLPs in the range of 5 µg to 10mg/dose for women and half this dose for children.

In a further aspect of treatment, the pharmaceutical compositions comprise VLP, pentameric complex, and/or soluble protein as separate or combined pharmaceutical compositions for a prime-boost regimen. Each component is used in the range of 10 µg to 10 mg/dose, respectively. The pharmaceutical composition of the combination of VLP and pentameric complex and/or soluble protein is in the range of 10 µg to 10 mg/dose of each component. Different ratios of the components are likewise possible. An average human of 70 kg is assumed to receive at least one single vaccination. Preferably a dosage regimen comprising 3 doses applied at 0, 8 and 24 weeks, optionally followed by a second vaccination round 12-24 months after the last immunization is chosen. Preferred routes of administration are subcutaneous (sc) and intramuscular (im) administration, but intradermal and intranasal are also suitable administrations.

The method of treatment of the invention is particularly important for solid organ, bone marrow and/or stem cell transplant recipients. For these patients a fast and effective CD8+ and CD4+ T-cell response is crucial. To address this topic the pharmaceutical composition of the present invention is in the range of 10 µg to 10 mg/dose. An average human of 70 kg is assumed to receive at least once a vaccination. Preferably a dosage regimen comprising 3 doses applied at 0, 2 and 4 weeks before transplantation, optionally followed by a second vaccination round 1, 4, 8 weeks after the transplantation is chosen.

Vaccines according to the invention comprise the recombinant virus-like particle, the pentameric complex and/or the soluble protein in aqueous solution, and optionally further viscosity-regulating compounds, stabilizing compounds and/or an adjuvant increasing the immunogenicity, as it is known in the state of the art.

### Examples

### Example 1: Construction of recombinant expression vectors RBT136-7 and RBT136-9

For the generation of expression constructs for the BEVS system the vector backbone pRBT136, containing elements for propagation in *E.coli* and yeast (e.g. *Saccharomyces cerevisae*) and for protein expression in insect cells, is used. For the various VLP variants different genes were chosen for providing the capsid, the tegument and surface proteins.

The genes are generated by gene synthesis with a specific optimization for *Spodoptera frugiperda* and chosen out of the following groups of the cytomegalovirus genes: first from the capsid compartment, e.g. pUL86, pUL85, pUL46, pUL48, pUL50, and pUL80.5 (UL80A); second from the tegument part, e.g. pUL83, pUL25, ppUL32, and pUL99, and third from the surface part, e.g. gpUL55 (gB), gpUL75 (gH), gpUL115 (gL), gpUL74 (gO), gpUL100 (gM), gpUL73 (gN), gpUL128, gpUL130, and gpUL131A. In order to build the HCMV capsid multiple gene combinations of pUL86, pUL85, pUL46, and pUL80a, optionally in combination with the functional genes pUL48a and pUL50, were used. The tegument chosen is pUL83 whereas the surface was prepared from gpUL55 (gB), gpUL75 (gH), gpUL115 (gL), gpUL74 (gO), gpUL128, gpUL130, and gpUL131A.

The parallel assembly of pUL86-pUL85-pUL50-pUL48-pUL46-pUL83-pUL80.5-gpUL74 (gO) into one expression vector as well as the assembly of gpUL75 (gH)-gpUL115 (gL)-gpUL128-gpUL130-gpUL131A in a second expression vector was conducted by homologous recombination of PCR products representing various expression cassettes of the type promoter-gene-terminator. The vector backbone pRBT136 contains an origin of replication for *E.coli* and yeast, the polh and p10 promoters for expression in insect cells, the terminator SV40 and HSVtk, several resistance markers (ampicillin, gentamycin), transposon sites (Tn) and a multiple cloning site (MCS). The expression cassette containing promoter - UL86 - terminator was PCR amplified at the 5' site with the primer RBT155 (TGATTTGATAATAATTCTTATTTAAC, SEQ ID NO:63) and at the 3' site with a 35-40nt overhang (GGCTAGCTTTGTTTAACTTTAAGAAGGAGATACATCTAGA, SEQ ID NO:64). For homologous recombination with the expression cassette containing promoter - UL85 - terminator, this part was PCR amplified at the N-terminal end with the complementary sequence of the 35-40nt overhang at the C-terminal end of the previous PCR product. The homologous recombination took place in yeast (*Saccharomyces cerevisiae*): An overnight culture was centrifuged at 500xg at 25°C for 5 min, the supernatant was aspirated and the pellet washed with water and with Tris-EDTA-LiOAc buffer. The vector, linearized with EcoRI and Hindlll (1 µl), salmon sperm DNA (8 µl) and the PCR products (1-4 µl) representing the expression cassettes were added to 50 µl washed yeast, incubated for 30 min at 30°C, followed by a heat shock at 42°C, and plated on a respective amino acid drop out agar plate without uracil, and incubated for 3 days. The resulting vector was isolated from the yeast cell by freeze (liquid nitrogen, 2 min) - thaw (95°C, 1 min) - lysis. After addition of the same volume chloroform the vector was precipitated with the double volume of ethanol. The precipitated vector (5 µl) was added to 50 µl competent XL-1 blue (Stratagene) followed by a transformation according to the manufacturer's protocol: Incubation on ice for 30 min, followed by a heat shock at 42°C for 45 sec, a 2 min cold shock at 4°C, addition of 200 µl 2YT medium, and a 1 h incubation at 220 rpm at 37°C. The culture was then plated on 2YT agar plates containing 100 µg ampicillin and 100 µg gentamycin. The grown clones were verified by PCR screening, analytical digests and sequencing to prove the presence of all assembled genes.

The genes UL83 and UL80.5 were assembled into the vectors pRBT4 and combined by cre-lox recombination with pRBT136 containing pUL86-pUL85-pUL50-pUL48-pUL46-gpUL74 (gO). 1 µl cre-recombinase was added to 500 ng of pRBT5 and pRBT136 in a 10 µl total volume and incubated for 30 min at 37°C. After a 10 min heat inactivation at 70°C a transformation in XL-1 blue competent cells was performed as described above. The clones were verified by PCR screening, analytical digest and sequencing. The sequenced plasmids were used for the generation of the corresponding baculovirus genome (bacmid). The genes of interest were transferred by site-specific homologous recombination via Tn7 directed transposition into competent DH10MB cells. 10 ng sequenced plasmid was added to 100 µl competent DH10MB cells and incubated for 30 min at 4°C. After a heat shock at 42°C for 45 sec, a 2 min cold shock at 4°C, addition of 400 µl 2YT medium and a 4 h incubation at 37°C at 220 rpm, a 1:10 dilution was plated on appropriate 2YT agar plates containing the antibiotics according to the resistance markers and IPTG/X-gal for blue-white screening. Correct clones were selected, expanded and DNA isolated using the Birnboim & Doly method (Nucleic Acids Res. 1979; 7(6):1513-23). Four correct bacmid clones (1 µg of each) were selected for initial transfection of insect cells (Sf9, 1x10⁶ cells/6-well) using the reagent Fugene (3 µl, Roche, Switzerland) to generate the recombinant seed virus (V₀). Two consecutive amplifications of this virus were performed using a multiplicity of infection (MOI) of 0.05 pfu/cell in a 200 ml culture for the generation of master seed virus (V₁) and working seed virus (V₂). The infective titer of the working seed virus was determined by a classical plaque assay. Virus dilutions (10⁵ to 10⁸ in a volume of 1 ml) were added to 1x10⁶ cells/6-well Sf9 cell culture, and incubated for 1 h at 27°C. After aspiration of the virus, the cells were overlayed with 4% agarose in Sf900-1.3 medium, followed by an incubation at 27°C in a humidified chamber. The infective titer of the different constructs were in the range of 1-5x10⁷ pfu/cell.

The production parameters such as cell line, cell count at infection (CCI), amount of recombinant virus inoculum (multiplicity of infection, MOI) and time of harvest (TOH) were determined in 50 ml bioreactors (Cultiflask, Sartorius Stedim) by infection of a 20 ml insect cell culture. A matrix of different CCIs (0.5; 1, 2 x10⁶ cells/ml), MOIs (0.1, 0.4, 1, 2, 4) and time of harvest (TOH) was conducted for 7 days to determine the best production parameters in respect of yield (Ries, C., John C., Eibl R. (2011), A new scale down approach for the rapid development of Sf21/BEVS based processes - a case study. In Eibl R., Eibl D. (Editors): Single-use technology in Biopharmaceutical Manufacture, 207-213, John Wiley & Sons, Hoboken, New Jersey). The production was controlled by daily sampling, determining cell count and viability. The samples were analysed by immunoblotting and ELISA. The best production parameters in respect of yield evaluated as highest intensity (Dot blot) and highest OD (ELISA) were reached 3 days post infection (p.i.) with a cell count of infection (CCI) of 2x10⁶ cells/mL and a MOI of 0.2 of each virus used for co-infection.

For the immunoblotting 100 µl of the cell culture samples were subjected to a nitrocellulose membrane (BioRAD) by filtration using a dot-blot chamber (BioRAD). After blocking unspecific binding sites for 30 min with 5% non-fat dry-milk TrisCl-Tween20 (0.1%) solution, the membrane was incubated over night at 4°C with antibodies against the tegument protein UL83. Protein detection was performed with NBT/BCIP (ThermoFisher) solution after incubation with an alkaline-phosphatase coupled secondary anti-mouse antibody (Cell signaling).

For the ELISA assay all collected cell culture samples were diluted 1:10 in 100 µl/well coating buffer (0.1 M Na₂HPO₄, pH 9) in a 96-well pre-absorbed ELISA plate (Nunc) and incubated over night at 4°C. Afterwards the plate was washed 3x with 195 µl/well wash buffer (1xPBS, 0.05% Tween 20) followed by a 1 h blocking at room temperature with 195 µl/well 3% BSA in 1x PBS solution. After 3 washing steps the specific antibodies in a concentration of 1 µg/ml in 3%BSA, 1x PBS, 0.05% Tween 20, pH 7 were added (100 µl/well) and incubated for 1 h at room temperature. A mouse-anti- gH (Santa Cruz) antibody was used for the detection of the surface protein gH whereas the tegument protein UL83 was verified with a mouse-anti-UL83 antibody (Virusys). After 3 washing steps a 1 h incubation with the appropriate secondary antibody (anti-mouse-lgG-HRP, 1:1000 dilution in 3%BSA, 1x PBS, 0.05% Tween20, pH 7) was conducted. The binding of the specific antibodies (UL83, gH) to the VLP was detected using 100 µl/well TMP substrate reagent (BD Biosciences, San Diego, USA; according to manufacturer's protocol), thereafter the reaction was stopped after 3-15 min with 100 µl 1 M HCl, followed by OD measurement at 450 nm in a microplate reader.

The following expression vectors (pRBT136-x) for generation of CMV-VLP variants were processed as described in Example 1.
Abbreviations: c: consensus sequence; H: His-tag; SH: Streptavidin-His-tag; V: VR1814, pcl: precission protease, pclI:precission and TEV protease, DT: dimerization tool. For the description of the genes in Table 1 the shorter HCMV nomenclature (gB, gH, gL, gO as well as "UL" without prefix and UL48 without suffix "A") is used.

**Table 1**

| Vector variant (pRBT 136-x) SEQ ID | Contained genes | Variant with dimerisation tool (DT) at gH and capsid protein (gag or UL86) |
|---|---|---|
| 1 | UL86, Towne | |
| 2 | UL86-UL83-UL80.5, Towne | |
| 3 | UL86-UL85-UL83-UL80.5, Towne | |
| 4 | UL86-UL85-UL50-UL48A-UL46, Towne | |
| 5 | UL86-UL85-UL50-UL48A-UL46-UL83-UL80.5, Towne | |
| 6 | U L86-U L85-U L50-U L48A-U L46-U L74, Towne | |
| 7 | UL86-UL85-UL50-UL48A-UL46-UL74-UL83-UL80.5, Towne | |
| 8 | gB, Towne | |
| 9 | gH-gL-UL128-UL130-UL131A, Towne | gH (DT; Towne) |
| 10 | gH-gL-UL128(c)-UL130-UL131A, Towne | gH (DT; Towne) |
| 11 | gH-gL(H)-UL128-UL130-UL131A, Towne | |
| 12 | gH-gL(H)-UL128(c)-UL130-UL131A, Towne | gH (DT; Towne) |
| 13 | gH-gL(SH)-UL128-UL130-UL131A, Towne | |
| 14 | gH-gL(SH)-UL128(c)-UL130-UL131A, Towne | gH (DT; Towne) |
| 15 | gH-gL-UL128(Towne)-UL130(V)-UL131A(V) | |
| 16 | gH-gL-UL128(c)-UL130(V)-UL131A(V) | |
| 17 | gL-UL128-UL130-UL131A-gB, Towne | |
| 18 | gL-UL128-UL130-UL131A-gB-gH, Towne | gH (DT; Towne) |
| 19 | gL-UL128-UL130-UL131A-gB-gH-UL83, Towne | |
| 20 | Gag | |
| 21 | Gag-dimerization tool (DT) | |
| 22 | Gag-UL83 | |
| 23 | gH(DT)-gL-UL128-UL130-UL131A, Towne | |
| 24 | gH (DT)-gL-UL128(c)-UL130-UL131A, Towne | |
| 25 | gH(DT)-gL(SH)-UL128-UL130-UL131A, Towne | |
| 26 | gH (DT)-gL(SH)-UL128(c)-UL130-UL131A, Towne | |
| 27 | gL-UL128-UL130-UL131A-gB-gH(DT), Towne | |
| 28 | gL-UL128-UL130-UL131A-gB-gH-gag, Towne | gH (DT; Towne), gag (DT) |
| 29 | gL-UL128(c)-UL130-UL131A-gB(T)-gH (T)-gag | gH (DT), gag (DT) |
| 30 | gL-UL128(c)-UL130(V)-UL131A(V)-gB(T)-gH(T)-gag | gH (DT), gag (DT) |
| 31 | gL-UL128-UL130-UL131A-gB-gH-gag-UL83, Towne | |
| 32 | gL-UL128(c)-UL130-UL131A-gB-gH-gag-UL83, Towne | |
| 33 | gL-UL128(c)-UL130(V)-UL131A(V)-gB(T)-gH(T)-gag-UL83(T) | |
| 34 | gag-UL83, Towne | gag (DT) |
| 35 | UL86-UL85-UL50-UL48A-UL46-UL83-UL80.5, Towne | UL86 (DT) |
| 36 | UL128-UL130-UL131A- gL-gB-gH-UL86-UL85, Towne | gH (DT), UL86 (DT) |
| 37 | UL128 (c)-UL130-UL131A- gL-gB-gH-UL86-UL85, Towne | gH (DT), UL86 (DT) |
| 38 | UL128 (c)-UL130(V)-UL131A(V)- gL-gB-gH-UL86-UL85, Towne | gH (DT), UL86 (DT) |
| 39 | UL128-UL130-UL131A- gL-gB-gH-UL86-UL80.5, Towne | gH (DT), UL86 (DT) |
| 40 | UL128(c)-UL130-UL131A- gL-gB-gH-UL86- UL80.5, Towne | gH (DT), UL86 (DT) |
| 41 | UL128 (c)-UL130(V)-UL131A(V)- gL-gB-gH-UL86-UL80.5, Towne | gH (DT), UL86 (DT) |
| 42 | UL128-UL130-UL131A- gL-gB-gH-UL86-UL80.5-UL83-UL85, Towne | gH (DT), UL86 (DT) |
| 43 | UL128 (c)-UL130-UL131A- gL-gB-gH-UL86-UL80.5-UL83-UL85, Towne | gH (DT), UL86 (DT) |
| 44 | UL128(c)-UL130(V)-UL131A(V)-gL-gB-gH- UL86-UL80.5-UL83-UL85, Towne | gH (DT), UL86 (DT) |
| 45 | UL128-UL130-UL131A- gL-gB-gH-UL80.5-UL83-UL86-UL85-UL50-UL48A-UL46, Towne | gH (DT), UL86 (DT) |
| 46 | UL128(c) -UL130-UL131A- gL-gB-gH-UL83-UL80.5-UL86-UL85-UL50-UL48A-UL46, Towne | gH (DT), UL86 (DT) |
| 47 | UL128(c) -UL130(V)-UL131A(V)- gL-gB-gH-UL83-UL80.5-UL86-UL85-UL50-UL48A-UL46, Towne | gH (DT), UL86 (DT) |
| 48 | UL74-UL86-UL83-UL80.5-UL85, Towne | UL86 (DT) |
| 49 | UL128-UL130-UL131A- gL-gB-gH-UL86-UL85-UL83-UL80.5-UL74, Towne | gH (DT), UL86 (DT) |
| 50 | UL128(c) -UL130-UL131A- gL-gB-gH-UL86-UL85-UL83-UL80.5-UL74, Towne | gH (DT), UL86 (DT) |
| 51 | UL128(c) -UL130(V)-UL131A(V)- gL-gB-gH-UL86-UL85-UL83-UL80.5-UL74, Towne | gH (DT), UL86 (DT) |
| 52 | UL74-UL86-UL85-UL50-UL48A-UL46-UL83-UL80.5, Towne | UL86 (DT) |
| 53 | UL128-UL130-UL131A- gL-gB-gH-UL86-UL85-UL83-UL80.5-UL74-UL50-UL48A-UL46, Towne | gH (DT), UL86 (DT) |
| 54 | UL128(c) -UL130-UL131A- gL-gB-gH- UL86-UL85-UL83-UL80.5-UL74-UL50-UL48A-UL46, Towne | gH (DT), UL86 (DT) |
| 55 | UL128(c)-UL130(V)-UL131 A(V)-gL-gB-gH-UL86-UL85-UL83-UL80.5-UL74-UL50-UL48A-UL46, Towne | gH (DT), UL86 (DT) |
| 56 | UL128-UL130-UL131A- gL-gB-gH-UL86-UL85-UL83- - UL50-UL48A-UL46, Towne | gH (DT), UL86 (DT) |
| 57 | UL128(c)-UL130-UL131A- gL-gB-gH-UL86-UL85-UL83--UL50-UL48A-UL46, Towne | gH (DT), UL86 (DT) |
| 58 | UL128(c)-UL130(V)-UL131A(V)- gL-gB- gH-UL86-UL85-UL83-UL50-UL48A-UL46, Towne | gH (DT), UL86 (DT) |
| 59 | gH (without membrane anchor, His)-gL-UL128-UL130-UL131A, Towne | |
| 60 | gH (without membrane anchor,SH)-gL-UL128-UL130-UL131A, Towne | |
| 61 | gH (without membrane anchor, H-pcl)-gL-UL128-UL130-UL131A, Towne | |
| 62 | gH (without membrane anchor, H- pclI)-gL-UL128-UL130-UL131A, Towne | |
| 67 | UL86-UL85 | UL86 (DT) |
| 68 | UL73-UL86-UL80.5-UL83-UL85, Towne | UL86 (DT) |
| 69 | UL86-UL80.5-UL83-UL85-UL73, Towne | UL86 (DT) |
| 70 | UL100-UL86-UL80.5-UL83-UL85, Towne | UL86 (DT) |
| 71 | UL86-UL80.5-UL83-UL85-UL100, Towne | UL86 (DT) |
| 72 | UL100-UL73, Towne | |
| 73 | UL100-UL73-UL86-UL80.5-UL83-UL85-, Towne | UL86 (DT) |
| 74 | UL86-UL83-UL80.5-UL85-UL100-UL73, Towne | UL86 (DT) |
| 75 | UL74-UL86-UL83-UL80.5-UL85-UL73, Towne | UL86 (DT) |
| 76 | UL74-UL86-UL83-UL80.5-UL85-UL100, Towne | UL86 (DT) |
| 77 | UL74-UL86-UL83-UL80.5-UL85-UL100-UL73, Towne | UL86 (DT) |
| 78 | U L86-U L85-U L48A | UL86 (DT) |
| 79 | UL86-UL85-UL48A-UL100 | UL86 (DT) |
| 80 | UL86-UL85-UL48A-UL83 | UL86 (DT) |
| 81 | UL86-UL85-UL48A-UL83-UL100 | UL86 (DT) |
| 82 | UL86-UL85-UL48A-UL83-UL100-UL73 | UL86 (DT) |
| 83 | UL74-UL86-UL85-UL48A-UL83-UL100 | UL86 (DT) |
| 84 | UL74-UL86-UL85-UL48A-UL83-UL100-UL73 | UL86 (DT) |
| 85 | UL128-UL130-UL131 A-gL-gB-gH-UL86-UL85-UL83-UL80.5-UL50-UL48A-UL46, Towne | UL86 (DT) |
| 86 | UL128(c)-UL130-UL131A- gL-gB-gH-UL86-UL85-UL83-UL80.5-UL50-UL48A-UL46, Towne | UL86 (DT) |
| 87 | UL128(c)-UL130(V)-UL131A(V)- gL-gB-gH-UL86-UL85-UL83-UL80.5-UL50-UL48A-UL46, Towne | UL86 (DT) |
| 88 | MoMULV gag | DT |
| 89 | MoMULVgag-UL83 | DT |
| 90 | UL74-UL86-UL85-UL48A-UL83-UL-100gL-UL128-UL130-UL131A-gB-gH, Towne | UL86(DT), gH(DT) |
| 91 | UL86-UL85-UL48A-UL83-UL100-gL-UL128(c)-UL130(V)-UL131A(V)-gB-gH, Towne | UL86(DT), gH(DT) |

### Example 2: Expression vectors pRBT136CMV-1; pRBT136CMV-8 and pRBT136CMV-20

To obtain the expression vectors pRBT136CMV-1; pRBT136CMV-8 and pRBT136CMV-20 each gene of interest, namely UL86, UL55 (gB), and gag, was subcloned from a pBluescript vector by BamHI (5') and HindIII (3') digestion and ligation into the backbone pRBT136 cut with the same enzymes. The ligation was conducted with 2 µg cut pRBT136, 10 µg fragment and 1 µl T4-ligase (NEB) in a total volume of 20 µl, followed by a transformation in competent XL-1 blue cells as described in Example 1.

### Example 3: Expression construct for capsid variant pRBTCMV-2

To obtain the expression construct for capsid variant pRBTCMV-2 for the generation of CMV-VLP an expression cassette containing UL86 and another with UL83 were assembled in parallel as described in Example 1. The integration of the UL80.5 expression cassette was done by ligation of a PCR product using as forward primer RBT204 (TGCTGCCCACCGCTGAGCAATAACTATCATAACCCCCGGAATATTAATA GATCATGG, SEQ ID NO:65) and a reverse primer RBT189 (GTAGCGTCGTAAGCTA ATACG, SEQ ID NO:66) by ligation as described in Example 2.

### Example 4: Expression construct for capsid variant pRBTCMV-3

To generate the expression construct for capsid variant pRBTCMV-3, the plasmid of variant pRBTCMV-2 was cut with the restriction enzyme AvrlI. The expression cassette with the gene UL85 was amplified with the primers pRBT526 (5') and pRBT189 (3') and introduced in the plasmid pRBTCMV-2 by homologous recombination as described in Example 1.

### Example 5: Expression, purification and characterization of the CMV-VLP variant 5

According to the protein expression parameters defined after systematic optimization (Example 1) the virus-like particles (VLP) comprising UL86-UL85-UL50-UL48-UL46-UL83-UL80.5-UL74- gH-gL-UL128-UL130-UL131A are produced in disposable 1 L shake flasks (culture volume 300 mL) in fall army worm *Spodoptera frugiperda* cells (Sf9) by co-infection of 2 baculoviruses (SEQ ID NO:5 and SEQ ID NO:12) containing each multiple genes. The production parameters were as follows: Initial cell count at infection (CCI) of 2x10⁶ cells/ml, a multiplicity of infection (MOI) of 0.4 pfu/ml, for each virus 0.2 pfu/ml, incubation at 27°C at 100 rpm. Harvest took place at day 3 post infection (p.i.) at a viability around 80%. The production was controlled by daily sampling, determining cell count and viability. The VLP containing supernatant was concentrated 3x using tangential-flow-filtration with a Hydrosart Sartocon Slice200 cassette with a molecular weight cut off (MWCO) of 100 kDa and a filter area of 0.02 m² (Sartorius Stedim) at a flow rate of 80 ml/min and a transmembrane pressure between 1-1.5 bar. The retentate was subjected to purification by a 2-step glycerol-tartrate gradient ultracentrifugation. In a first step 25 ml retentate were overlayed onto a 6 mL 30% sucrose cushion (w/v in 50 mM Tris, 100 mM NaCl, pH 7.4) and pelleted for 1.5 h at 16000 rpm and 16°C using a SW28 rotor. The pellet was resuspended in 0.5 ml TN-buffer (50 mM Tris, 100 mM NaCl, pH 7.4). A glycerol-tartrate gradient (30% glycerol mixed with 15-35% tartrate, 12 ml volume) was prepared, overlayed with the 0.5 ml pre-purified material and centrifuged for 16 h at 24000 rpm and 16°C using a SW41 rotor. The gradient was fractionated from top in 0.9 ml fractions and the purified VLPs were analysed by biochemical methods. 150 µl of different gradient fractions were loaded onto a 4-12% Bis-Tris NuPAGE gel (Invitrogen) according to the manufacturer's protocol, run for 15 min at 150 V and for 45 min at 180 V using MOPS running buffer. The gels were stained over night with SimplyBlue SafeStain reagent (Invitrogen) and destained with water.

For immunoblotting the proteins were transferred onto a nitrocellulose membrane (BioRAD) at 19 V for 1 h using a semi-dry apparatus (BioRAD). After blocking unspecific binding sites for 30 min with 5% non-fat dry-milk TrisHCl-Tween20 (0.1%) solution, the membrane was incubated over night at 4°C with antibodies against the specific proteins such as the tegument protein pUL83. Protein detection was performed with NBT/BCIP (ThermoFisher) solution after incubation with an alkaline-phosphatase coupled secondary anti-mouse antibody (Cell Signaling).

Total protein is determined *via* a Bradford assay adapted to a 96-well plate format (BCA, Pierce). 20 µl of unknown or standard sample was diluted in 180 µl of buffer. Serial 2-fold dilutions are made to the standard in triplicate (pure bovine serum albumin) or unknown samples. 100 µl of 2x stock Bradford reagent (Pierce) was added per well. The plate is then mixed and absorbance was measured at 595 nm in a microtiter plate reader. The CMV-VLPs contained in the different fractions obtained by fractionating the glycerol-tartrate gradient are further analysed by investigating the binding of a specific antibody against the tegument UL83 (mouse-anti-UL83, Virusys) and surface protein gH (mouse-anti-gH, Santa Cruz). Therefore the different gradient fractions were 1:10 diluted in 100 µl/well coating buffer (0.1 M Na₂HPO₄, pH 9) in a 96-well pre-absorbed ELISA plate and incubated over night at 4°C. Afterwards the plate was washed 3x with 195 µl/well wash buffer (1xPBS, 0.05% Tween 20) followed by a 1 h blocking at room temperature with 195 µl/well 3%BSA in 1x PBS solution. After 3 washing steps the specific antibody, the anti-surface antibody (anti-gH,) as well as the anti-tegument antibody (anti-UL83) in a concentration of 1 µg/ml in 3%BSA, 1x PBS, 0.05% Tween 20, pH 7 was added (100 µl/well) and incubated for 1 h at room temperature followed by further 3 wash steps. For detection a 1 h incubation with the appropriate secondary antibody (anti-mouse-IgG-HRP, 1:1000 dilution in 3%BSA, 1x PBS, 0.05% Tween20, pH 7) was conducted. The binding of the specific antibody to the VLP was detected using 100 µl/well TMP substrate reagent (BD Biosciences, San Diego, USA; according to manufacturer's protocol), wherefore the reaction is stopped after 3-15 min with 100 µl 1 M HCl, followed by OD measurement at 450 nm in a microplate reader.

The following Table 2 is an overview of the CMV-VLP variants which were expressed, purified and characterized as described in Example 5. The genetic constructs described in Table 1 were quality controlled by sequencing before they were used for further manipulation such as generation of the corresponding baculoviruses. The integrity of the generated baculoviruses is quality controlled by PCR based transgene control for presence of each single gene. The most important properties for manufacturing and regulatory approval such as yield and co-localization of the proteins, especially of the immunogenic surface proteins of these CMV VLPs are stated in Table 2.

**Table 2**

| Manufactured VLPs | capsid | tegument | surface | properties |
|---|---|---|---|---|
| 1 | 86, towne | - | gB-gH-gL-128-130-131, towne | VLP amount low |
| 2 | 86-85-80.5, towne | 83, towne | gB-gH-gL-128-130-131, towne | VLP amount low |
| 3 | 86-80.5, towne | 83, towne | gB-gH-gL-128-130-131, towne | VLP amount low |
| 4 | 86-85-50-48-46-80.5, towne | 83, towne | gH-gL-128-130-131, towne | good co-localization |
| 5 | 86-85-50-48-46-80.5, towne | 83, towne | gO-gH-gL-128-130-131, towne | good co-localization |
| 6 | 86-85-50-48-46-80.5, towne | 83, towne | gB-gH-gL-130-131 towne; 128 consensus sequence | good co-localization |
| 7 | 86-85-50-48-46-80.5, towne | 83, towne | gO-gB-gH-gL-130-131 towne; 128 consensus sequence | good co-localization |
| 8 | 86-85-80.5, towne | 83, towne | gB-gH-gL-130-131 towne; 128 consensus sequence | good co-localization |
| 9 | 86-85-80.5, towne | 83, towne | gO-gB-gH-gL-130-131 towne; 128 consensus sequence | good co-localization |
| 10 | 86-85-80.5, towne | 83, towne | gB-gH-gL-128 towne, 130-131 VR1814 strain | VLP amount medium |
| 11 | 86-85-80.5, towne | 83, towne | gO-gB-gH-gL-128 towne, 130-131 VR1814 strain | good co-localization |
| 12 | 86-85-80.5, towne | 83, towne | gB-gH-gL-towne, 128 consensus, 130-131 VR1814 strain | good co-localization |
| 13 | 86-85-80.5, towne | 83, towne | gO-gB-gH-gL-towne, 128 consensus, 130-131 VR1814 strain | good co-localization |
| 14 | 86-85-80.5, towne | 83, towne | gB-gH-gL-128 towne, 130-131 VR1814 strain | good co-localization |
| 15 | 86-80.5, towne | 83, towne | gO-gB-gH-gL-128 towne, 130-131 VR1814 strain | good co-localization |
| 16 | 86-80.5, towne | | gB-gH-gL-towne, 128 consensus, 130-131 VR1814 strain | good co-localization |
| 17 | 86-80.5, towne | | gO-gB-gH-gL-towne, 128 consensus, 130-131 VR1814 strain | good co-localization |
| 18 | gag | - | gH-gL-128-130-131, towne | good co-localization |
| 19 | gag | - | gB-gH-gL-128-130-131, towne | good co-localization |
| 20 | gag | 83, towne | gB-gH-gL-128-130-131, towne | Very good colocalization |
| 21 | gag | - | gB-gH-gL-128 towne, 130-131 VR1814 strain | good co-localization |
| 22 | gag | - | gO-gB-gH-gL-128 towne, 130-131 VR1814 strain | good co-localization |
| 23 | gag | - | gB-gH-gL-towne, 128 consensus, 130-131 VR1814 strain | good co-localization |
| 24 | gag | - | gO-gB-gH-gL-towne, 128 consensus, 130-131 VR1814 strain | good co-localization |

### Example 6: Expression, purification and characterization of the pentameric CMV complex.

According to the protein expression parameters defined based on systematic optimization (Example 1 for the VLPs) the pentameric CMV complex comprising gpUL75 (gH-His)-gpUL115 (gL)-gpUL128-gpUL130-gpUL131A is produced in disposable 2 L shake flasks (culture volume 700 ml) in fall army worm *Spodoptera frugiperda* cells (Sf9) by co-expression from a single baculovirus (SEQ ID NO:59). The production parameters were as follows: Initial cell count at infection (CCI) of 2x10⁶ cells/ml, a multiplicity of infection (MOI) of 0.25 pfu/ml, incubation at 27°C at 100 rpm. Harvest took place at day 3 post infection (p.i.) at a viability around 80%. The production was controlled by daily sampling, determining cell count and viability. The complex containing supernatant was loaded on 2x 5 ml HisTrap colums (GE Healthcare). The complex was purified using a linear gradient from zero to 500 mM imidazole over 50 column volumes (CV, equivalent to 500 ml. The different chromatographic fractions were analysed by biochemical methods. 150 µl of different fractions were precipitated with acetone, resuspended in 30 µl 20 mM Tris,150 mM NaCl buffer, pH 7.4. For loading onto a 4-12% Bis-Tris NuPAGE gel (Invitrogen) 4x loading dye was added according to the manufacturer's protocol, followed by the electrophoreses for 15 min at 150 V and for 45 min at 180 V using MOPS running buffer. The gels were stained over night with SimplyBlue SafeStain reagent (Invitrogen) and destained with water. For concentration and further purification a 2^{nd} IMAC chromatography was done using a 1 ml HisTrap column by a linear gradient over 50 column volumes from 0-500 mM imidazole. The different fractions were analysed like the 1^{st} IMAC step by Coomassie stained SDS-PAGE and immunoblotting using an anti-His antibody. All complex containing fractions were pooled, concentrated to a final volume of 5 ml using a 50 kDa cut off Amicon filter unit (Millipore) and loaded for a final purification step on a size exclusion column (XK16/69, Superdex200pg) and analysed by SDS-PAGE followed by Coomassie staining and immunoblotting using an anti-His antibody. Total protein is determined *via* a Bradford assay adapted to a 96-well plate format (BCA, Pierce). 20 µl of unknown or standard sample was diluted in 180 µl of buffer. Serial 2-fold dilutions are made to the standard in triplicate (pure bovine serum albumin) or unknown samples. 100 µl of 2x stock Bradford reagent (Pierce) was added per well. The plate is then mixed and absorbance was measured at 595 nm in a microtiter plate reader. The pentameric CMV complex contained in the different fractions from the chromatographic based purification is further analysed by investigating the binding of a specific antibody against the His-tag added to gpUL75 (gH, mouse-anti-His, AbD Serotec) and the gpUL75 itself (gH, mouse-anti-gH, Santa Cruz). Therefore the different samples were 1:10 diluted in 100 µl/well coating buffer (0.1 M Na₂HPO₄, pH 9) in a 96-well pre-absorbed ELISA plate and incubated over night at 4°C. Afterwards the plate was washed 3x with 195 µl/well wash buffer (1xPBS, 0.05% Tween 20) followed by a 1 h blocking at room temperature with 195 µl/well 3%BSA in 1x PBS solution. After 3 washing steps the specific antibody, the anti-His antibody (anti-His) as well as the anti-gpUL75 (anti-gH) in a concentration of 1 µg/ml in 3%BSA, 1x PBS, 0.05% Tween 20, pH 7 was added (100 µl/well) and incubated for 1 h at room temperature followed by further 3 wash steps. For detection a 1 h incubation with the appropriate secondary antibody (anti-mouse-IgG-HRP, 1:1000 dilution in 3%BSA, 1x PBS, 0.05% Tween20, pH 7) was conducted. The binding of the specific antibody to the pentameric complex was detected using 100 µl/well TMP substrate reagent (BD Biosciences, San Diego, USA; according to manufacturer's protocol), wherefore the reaction is stopped after 3-15 min with 100 µl 1 M HCl, followed by OD measurement at 450 nm in a microplate reader.

### Example 7: Visual verification of generated assemblies by electron microscopy

To verify the shape of the purified VLPs, electron microscopy was performed. A 10 µl drop of VLP sample was incubated 10 min on a copper covered EM grid, stained with 5 µl phosphor tungsten acid for 5-10 min and de-stained 2 times with water for 1 min. The liquid on the grid was removed with a Whatman paper and the grid was transferred to the JEOL3000 microscope, investigated and pictures of the particles taken.

### Example 8: Binding assay the use of sandwich ELISA

A sandwich ELISA as binding assay was performed to determine the presence and accessibility of the surface proteins of different reVLPs (recombinant VLPs). Therefore a specific surface and/or tegument antibody in a concentration of 1 µg/ml in 3%BSA, 1x PBS, 0.05% Tween 20, pH 7 was added (100 µl/well) in coating buffer (0.1 M Na₂HPO₄ pH 9) in a 96-well pre-absorbed ELISA plate and incubated over night at 4°C. Afterwards the plate was washed 3x with 195 µl/well wash buffer (1xPBS, 0.05% Tween 20) followed by a 1 h blocking at room temperature with 195 µl/well 3% BSA in 1x PBS solution. After 3 washing steps the different VLPs in a 1:10 dilution in 3% BSA, 1x PBS, 0.05% Tween 20, pH 7 were added (100 µl/well) and incubated for 1 h at room temperature. After 3 washing steps the specific antibody, the anti-surface antibody (anti-gH, anti-gB) as well as the anti-tegument antibody (anti-UL83) in a concentration of 1 µg/ml in 3% BSA, 1x PBS, 0.05% Tween 20, pH 7 were added (100 µl/well) and incubated for 1 h at room temperature followed by further 3 wash steps. For detection a 1 h incubation with the appropriate secondary antibody (anti-mouse-lgG-HRP, 1:1000 dilution in 3% BSA, 1x PBS, 0.05% Tween 20, pH 7) was conducted. The binding of the specific antibody to the VLP was detected using 100 µl/well TMP substrate reagent (BD Biosciences, San Diego, USA; according to manufacturer's protocol). The reaction is stopped after 3-15 min with 100 µl 1 M HCl, followed by OD measurement at 450 nm in a microplate reader.

### Example 9: In vivo study in mice

For the *in vivo* study Balb/C mice were used in a prime-boost-boost regimen. Each group contained 8 mice and each mouse received 20 µg protein per injection. For the combination (CMV-reVLP + pentameric complex) a total amount of 40 µg protein was injected. Pre-immune sera were taken after 14 days quarantine of the mice (day 0). The first injection took place 10 days later followed by a booster injection at day 42. The first bleeding was done at day 49. The 2^{nd} booster injection was performed at day 61 followed by a further bleeding at day 70. The final bleeding took place at day 85 followed by the investigation of humoral and cellular immune response.

### Example 10: Humoral immune response based on neutralization assay.

The humoral immune response of the vaccine candidate based on the combination of a CMV-reVLP (vRBT-20) and the pentameric complex (SEQ ID: 59) was investigated by a neutralization assay of the mice sera from example 9 in comparison to sera from CMV negative and CMV positive human blood donors. A BAC (bacterial artificial chromosome)-reconstituted VR1814 strain carrying a GFP molecule for analytical reasons (fix-EGFP) was used for the infection of fibroblasts (MRC-5) and epithelial (ARPE-19) cells to visualize the neutralisation potential of the mouse sera from Example 9. 2x10⁴ cells / well were seeded into a 96 well plate in RPMI medium containing 10% FCS (fetal calf serum).

A serum pool of the 8 mice was generated and added in 2-fold serial dilutions (1:20 to 1:2560) in 100 µl/well RPMI/FCS medium. The above mentioned fix-EGFP VR1814 virus was added in a tissue culture infectious dose (TCID) of 1000 virus molecules/well which was determined in a pre-assay. The 96 well plates were incubated for 8 days at 37°C in a CO₂ controlled atmosphere. The determination of green cell was performed in a plate reader with the following parameters: fluorescein-filter [excitation 485/20, emission 530/25), bottom reading mode, time: 0.1 sec, 25x measurements/well after 96 h incubation. The neutralization potency was determined as the dilution of the sera able to show a 50% virus infection inhibition. The following controls were performed: cell control (cells + PBS), virus control (only infected cells) and 5 CMV positive and 5 CMV negative sera from human blood donors.

### Example 11: Cellular immune response based on EliSpot data (Multiplex assay).

At day 85 of the *in vivo* study (Example 9) mice were killed and the spleenocytes prepared for the analysis of 10 different cytokines. For the restimulation of the spleenocytes the CMV-reVLP as well as mixes of synthetic peptides were used. The following proteins were verified: HIVgag, pUL83, gpUL75, gpUL115, gpUL55, gpUL128, gpUL130 and gpUL131A. An epitope prediction of each protein was done using several bioinformatics algorithms. For each protein a mix of 4 peptides were generated for the restimulation of the spleenocytes. For HIVgag a commercially available peptide mix of 130 peptides (JPT, Berlin) was used. The cytokines (IFN-gamma, IL-1beta, IL-2, IL-4, IL-5, IL-6, IL-10, IL-12, gCMSF and TNFalpha) were investigated by a multiplex assay kit from Invitrogen according to the manufacturer's protocol.

### Example 12: Binding assays based on reaction of antibodies present in CMV positive blood serum

To characterize the different CMV VLPs and soluble complexes as part of a vaccine a double binding assay in a sandwich ELISA format was performed. The presence and accessibility of capsid, tegument and surface proteins of different reVLPs was investigated by a combination of purified monoclonal antibodies and antibodies present in a sera-mix of HCMV positive blood donors. Therefore the anti-CMV monoclonal antibodies such as anti-gH, anti-gB, anti-UL83, anti-UL86, anti-UL85, anti-UL130/UL131A, anti-gag, anti-His were diluted to a final concentration of 1µg/ml coating buffer (0.1 0.1 M Na₂HPO₄, pH 9) in a 96 well pre-absorbed ELISA plate (100µl/well) and incubated over night at 4°C. Afterwards the plate was washed 3x with 195 µl/well wash buffer (1x PBS, 0.05% Tween 20) followed by a 1 h blocking at room temperature with 195 µl/well 3% BSA in 1x PBS solution. After 3 washing steps the different CMV VLPs and soluble complex in a 1:10 dilution in 3% BSA, 1x PBS, 0.05% Tween 20, pH 7 were added (100 µl/well) and incubated for 1 h at room temperature. After 3 washing steps a mix of 4 CMV positive human sera was added in a 1:300 dilution in 3% BSA, 1x PBS, 0.05% Tween 20, pH 7 (100 µl/well) and incubated for 1 h at room temperature followed by further 3 wash steps. For detection a 1 h incubation with the appropriate secondary antibody (anti-human-IgG-HRP, 1:1000 dilution in 3% BSA, 1x PBS, 0.05% Tween 20, pH 7) was conducted. The binding of the antibodies present in the human sera to the proteins composing VLPs and soluble complex was detected using 100 µl/well TMP substrate reagent (BD Biosciences, San Diego, USA; according to manufacturer's protocol). The reaction is stopped after 3-15 min with 100 µl 1 M HCl, followed by OD measurement at 450 nm in a microplate reader. This kind of sandwich assay was chosen to verify the integrity of the CMV VLPs and the soluble complex based on the binding on one side to monoclonal antibodies and on the other side to antibodies present in CMV positive sera. The binding to human antibodies in the sera showed the accessibility of proteins of the CMV VLPs and soluble complex.

### Example 13: Characterization of different CMV-reVLPs and the soluble complex using blood serum from CMV positive donors

The different CMV-reVLP variants were produced as described in Example 5 and subjected to concentration by a sucrose cushion based ultracentrifugation. 25 ml cell culture supernatant was overlayed onto a 6 mL 30% sucrose cushion (w/v in 50 mM Tris, 100 mM NaCl, pH 7.4) and pelleted for 1.5 h at 20'000 rpm and 16°C using a SW28 rotor. The pellet was resuspended in 0.2-0.4 ml TN-buffer (50 mM Tris, 100 mM NaCl, pH 7.4). 50 µl of the resuspended pellet was loaded onto a 4-12% Bis-Tris NuPAGE gel (Invitrogen) according to the manufacturer's protocol, run for 15 min at 150 V and for 60 min at 180 V using MOPS running buffer. The gels were stained over night with SimplyBlue SafeStain reagent (Invitrogen) and de-stained with water.
For immunoblotting the proteins were transferred onto a nitrocellulose membrane (BioRAD) at 19 V for 1 h using a semi-dry apparatus (BioRAD). After blocking unspecific binding sites for 30 min with 5% non-fat dry-milk TrisCl-Tween 20 (0.1 %) solution, the membrane was incubated over night at 4°C with either the sera from the mice (pool of the 8 mice per group) or from the human blood donors mentioned in Example 10 in a 1:500 dilution with 5% non-fat-dry-milk TrisCl-Tween20 (0.1 %). Protein detection was performed with NBT/BCIP (ThermoFisher) solution after incubation with an alkaline-phosphatase coupled secondary anti-mouse antibody (Cell signaling).

## Claims

1. A recombinant virus-like particle comprising one or more capsid or capsid precursor proteins, 3 or more surface proteins from cytomegalovirus (CMV), and optionally one or more tegument proteins.

2. The recombinant virus-like particle according to claim 1 comprising one or more capsid or capsid precursor proteins, 5 or more surface proteins from cytomegalovirus (CMV), and optionally one or more tegument proteins.

3. The recombinant virus-like particle according to claim 1 or 2 comprising (i) one or more capsid proteins from a herpes virus, and one or more tegument proteins from human cytomegalovirus (HCMV); or
(ii) one or more capsid or capsid precursor proteins from a retrovirus; or
(iii) one or more capsid proteins from a herpes virus, 3 or more surface proteins from human cytomegalovirus (HCMV) and optionally one or more tegument proteins from human cytomegalovirus (HCMV).

4. The recombinant virus-like particle according to claim 3(i), wherein the herpesvirus is human cytomegalovirus HCMV.

5. The recombinant virus-like particle according to claim 3(ii), wherein the one or more capsid or capsid precursor proteins from a retrovirus is gag.

6. The recombinant virus-like particle according to any one of claims 1 to 5, wherein the cytomegalovirus surface proteins are selected from the group consisting of gpUL75 (gH), gpUL115 (gL), gpUL55 (gB), gpUL74 (gO), gpUL100 (gM), gpUL73 (gN), gpUL128, gpUL130, and gpUL131A.

7. The recombinant virus-like particle according to any one of claims 1 to 5, wherein the tegument proteins are selected from the group consisting of pUL83 and pUL32

8. A DNA encoding the proteins comprised in a virus-like particle according to anyone of claims 1 to 7.

9. A vector comprising DNA according to claim 8.

10. A baculovirus vector according to claim 91.

11. A host cell comprising a vector according to claim 9 or 10.

12. A vaccine comprising a recombinant virus-like particle according to any one of claims 1 to 7.

13. The vaccine of claim 12, further comprising the pentameric complex consisting of gpUL75, gpUL115, gpUL128, gpUL130 and gpUL131A; or a soluble CMV protein selected from the group consisting of gpUL75, gpUL115, gpUL55, gpUL74, gpUL100, gpUL73, gpUL128, gpUL130, and gpUL131A.

14. The vaccine of claim 12 or 13, further comprising a soluble CMV protein selected from the group consisting of pUL83, IE-1, pUL99, pUL91, pUL82, and pp150.

15. The vaccine of any one of claims 12 to 14 comprising CMV proteins from different CMV strains selected from the group of Towne, Toledo, AD169, Merlin, TB20, and VR1814 strains.

## Patentansprüche

1. Rekombinanter Virusartiger Partikel (VLP) umfassend einen oder mehrere Kapsidproteine oder Kapsidvorstufenproteine, 3 oder mehr Oberflächenproteine von dem Cytomegalovirus (CMV) und wahlweise ein oder mehrere Tegumentproteine.

2. Der rekombinante Virusartige Partikel nach Anspruch 1, umfassend ein oder mehrere Kapsidproteine oder Kapsidvorstufenproteine, 5 oder mehrere Oberflächenproteine von dem Cytomegalovirus (CMV) und wahlweise ein oder mehrere Tegumentproteine.

3. Der rekombinante Virusartige Partikel nach Anspruch 1 oder 2, umfassend (i) ein oder mehrere Kapsidproteine von einem Herpesvirus und ein oder mehrere Tegumentproteine von dem humanen Cytomegalovirus (CMV); oder
(ii) ein oder mehrere Kapsidproteine oder Kapsidvorstufenproteine von einem Retrovirus; oder
(iii) ein oder mehrere Kapsidproteine von einem Herpesvirus, 3 oder mehr Oberflächenproteine von dem humanen Cytomegalovirus (CMV) und wahlweise ein oder mehrere Tegumentproteine von dem humanen Cytomegalovirus (CMV).

4. Der rekombinante Virusartige Partikel nach Anspruch 3(i), wobei der Herpesvirus der humane Cytomegalovirus (HCMV) ist.

5. Der rekombinante Virusartige Partikel nach Anspruch 3(ii), wobei das ein oder mehrere Kapsidproteine oder Kapsidvorstufenproteine von einem Retrovirus ein gag-Gen (gruppenspezifisches Antigen) ist.

6. Der rekombinante Virusartige Partikel nach einem der Ansprüche 1 bis 5, wobei die Cytomegalovirus-Oberflächenproteine ausgewählt sind aus der Gruppe bestehend aus gpUL75 (gH), gpUL115 (gL), gpUL55 (gB), gpUL74 (gO), gpUL 100 (gM), gpUL73 (gN), gpUL128, gpUL130 und gpUL131A.

7. Der rekombinante Virusartige Partikel nach einem der Ansprüche 1 bis 5, wobei die Tegumentproteine ausgewählt sind aus der Gruppe bestehend aus pUL83 und pUL32.

8. Eine DNS, die für die Proteine kodiert, die von einem Virusartigen Partikel nach einem der Ansprüche 1 bis 7 umfasst sind.

9. Ein Vektor umfassend DNS nach Anspruch 8.

10. Ein Baculovirus-Vektor nach Anspruch 9.

11. Eine Wirtszelle umfassend einen Vektor nach Anspruch 9 oder 10.

12. Ein Impfstoff umfassend einen rekombinanten Virusartigen Partikel nach einem der Ansprüche 1 bis 7.

13. Der Impfstoff nach Anspruch 12, weiter umfassend den pentameren Komplex bestehend aus gpUL75, gpUL115, gpUL128, gpUL130 und gpUL131A oder ein lösliches CMV-Protein, ausgewählt aus der Gruppe bestehend aus gpUL75, gpUL115, gpUL55, gpUL74, gpUL100, gpUL73, gpUL128, gpUL130 und gpUL131A.

14. Der Impfstoff nach Anspruch 12 oder 13, weiter umfassend ein lösliches CMV-Protein, ausgewählt aus der Gruppe bestehend aus pUL83, IE-1, pUL99, pUL91, pUL82 und pp150.

15. Der Impfstoff nach einem der Ansprüche 12 bis 14, umfassend CMV-Proteine von verschiedenen CMV-Stämmen ausgewählt aus der Gruppe bestehend aus Towne, Toledo, AD169, Merlin, TB20 und VR1814 Stämmen.

## Revendications

1. Pseudo-particule virale (VLP) comprenant une ou plusieurs protéines de capside ou de précurseur de capside, 3 ou plus de protéines de surface du cytomégalovirus (CMV), et facultativement une ou plusieurs protéines de tégument.

2. Pseudo-particule virale selon la revendication 1 comprenant une ou plusieurs protéines de capside ou de précurseur de capside, 5 ou plus de protéines de surface du cytomégalovirus (CMV), et facultativement une ou plusieurs protéines de tégument.

3. Pseudo-particule virale selon la revendication 1 ou 2 comprenant (i) une ou plusieurs protéines de capside d'un virus de l'herpès et une ou plusieurs protéines de tégument du cytomégalovirus humain (HCMV); ou
(ii) une ou plusieurs protéines de capside ou de précurseur de capside d'un rétrovirus; ou
(iii) une ou plusieurs protéines de capside d'un virus de l'herpès, 3 ou plusieurs protéines de surface du cytomégalovirus humain (HCMV), et facultativement une ou plusieurs protéines de tégument du cytomégalovirus humain (HCMV).

4. Pseudo-particule virale selon la revendication 3(i), le virus de l'herpès étant le cytomégalovirus humain (HCMV).

5. Pseudo-particule virale selon la revendication 3 (ii), l'une ou plusieurs protéines de capside ou de précurseur de capside du rétrovirus étant un antigène GAG.

6. Pseudo-particule virale selon l'une quelconque des revendications 1 à 5, les protéines de surface du cytomégalovirus étant sélectionnées du groupe comportant la gpUL75 (gH), gpUL115 (gL), gpUL55 (gB), gpUL74 (gO), gpUL 100 (gM), gpUL73 (gN), gpUL128, gpUL130 et la gpUL131A.

7. Pseudo-particule virale selon l'une quelconque des revendications 1 à 5, les protéines de tégument étant sélectionnées du groupe comportant la pUL83 et la pUL32.

8. ADN codant les protéines comprises dans une pseudo-particule virale selon l'une quelconque des revendications 1 à 7.

9. Vecteur comprenant de l'ADN selon la revendication 8.

10. Vecteur du baculovirus selon la revendication 9.

11. Cellule hôte comprenant un vecteur selon la revendication 9 ou 10.

12. Vaccin comprenant une pseudo-particule virale selon l'une quelconque des revendications 1 à 7.

13. Vaccin selon la revendication 12, comprenant en outre le complexe pentamérique comportant la gpUL75, gpUL115, gpUL128, gpUL130 et la gpUL131A; ou une protéine CMV soluble sélectionnée du groupe comportant la gpUL75, gpUL115, gpUL55, gpUL74, gpUL100, gpUL73, gpUL128, gpUL130 et la gpUL131A.

14. Vaccin selon la revendication 12 ou 13, comprenant en outre une protéine CMV soluble sélectionnée du groupe comportant la pUL83, IE-1, pUL99, pUL91, pUL82 et la pp150.

15. Vaccin selon l'une quelconque des revendications 12 à 14 comprenant des protéines CMV de différentes souches CMV sélectionnées du groupe de souches Towne, Toledo, AD169, Merlin, TB20 et VR1814.
